# EUROPÄISCHE PATENTANMELDUNG

(11) **EP 2 606 726 A1**
(43) Veröffentlichungstag der Anmeldung: **26.06.2013**
(21) Anmeldenummer: 11194855.0
(22) Anmeldetag: 21.12.2011
(51) Int. Cl.: A01N 37/52, C07C 317/42, C07C 323/36, C07C 323/44, C07C 323/63

(54) **N-Arylamidine-substituierte trifluoroethylsulfid-Derivate als Akarizide und Insektizide**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft neue N-Arylamidine-substituierte Trifluoroethylsulfid-Derivate der Formel (I), 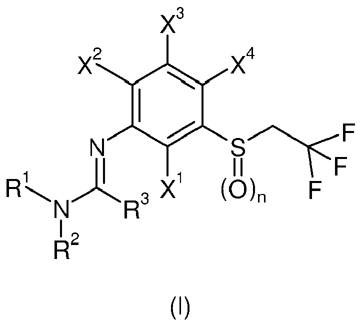 - in welcher X¹, X², X³, X⁴, R¹, R², R³, n die in der Beschreibung angegebenen Bedeutungen haben- deren Anwendung als Akarizide und Insektizide zur Bekämpfung tierischer Schädlinge und Verfahren sowie Zwischenprodukte zu ihrer Herstellung

## Beschreibung

Die vorliegende Erfindung betrifft neue N-Arylamidine-substituierte Trifluoroethylsulfid-Derivate, deren Anwendung als Akarizide und Insektizide zur Bekämpfung tierischer Schädlinge und Verfahren und Zwischenprodukte zu ihrer Herstellung.

Verschiedene substituierte N-Arylamidine und deren insektizide und akarizide Wirkung sind in der Literatur bereits beschrieben in WO 2007/131680.

Die gemäß den oben genannten Schriften bereits bekannten Wirkstoffe weisen bei ihrer Anwendung Nachteile auf, sei es, dass sie keine oder aber eine nur unzureichende insektizide und/oder akarizide Wirkung gegen tierische Schädlinge, insbesondere bei niedrigeren Aufwandmengen besitzen.

Aufgabe der vorliegenden Erfindung ist es daher, entsprechende N-Arylamidine-substituierte Trifluoroethylsulfid-Derivate bereitzustellen, welche als Insektizide und/oder Akarizide mit einer zufrieden stellenden insektiziden und/oder akariziden Wirkung gegen tierische Schädlinge, insbesondere bei niederigeren Aufwandmengen, mit einer hohen Selektivität und verbesserten Verträglichkeit in Nutzpflanzenkulturen eingesetzt werden können.

Es wurden nun neue Verbindungen der Formel (I) 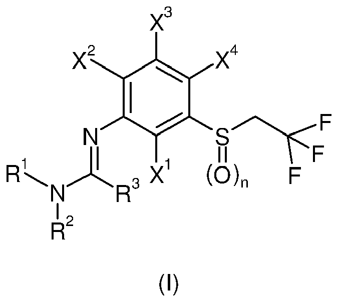 gefunden,
in welcher
n für die Zahl 0, 1 oder 2 steht,
X¹, X², X³, X⁴ unabhängig von einander für Wasserstoff, Halogen, Hydroxy, Amino, OCN, SCN, SF₅, Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl,Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Alkoxycarbonylalkyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl, Dialkylaminothiocarbonyl,
oder für gegebenenfalls substituiertes Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy Hetarylthio, Hetarylsulfinyl, Hetarylsulfonyl,
oder für gegebenenfalls substituiertes gesättigtes oder ungesättigtes Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl,
oder für NR⁴R⁵, wobei R⁴ und R⁵ unabhängig voneinander für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl, Alkoxycarbonyl stehen oder R⁴ und R⁵ gemeinsam mit dem N-Atom, an dem sie gebunden sind, einen gegebenenfalls substituierten und gegebenenfalls durch Heteroatome aus der Reihe O, S oder N unterbrochenen gesättigten oder ungesättigten fünf- bis achtgliedrigen Ring bilden können, stehen,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcarbonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes, gegebenenfalls durch Heteroatome aus der Reihe O, S oder N unterbrochenes Cycloalkyl, Cycloalkoxy, Cycloalkylalkyl oder Cycloalkylalkoxy steht,
oder, X² und X³, oder X³ und X⁴, bilden einen 5 oder 6-gliedrigen Ring mit den C-Atome an die sie gebunden sind aus, der gegebenenfalls subsitutiert ist und gegebenenfalls durch Heteroatome aus der Reihe O, S, N oder CO unterbrochen ist.
R³ für Wasserstoff, (C₂-C₈)Alkyl, Cyano, Halogenalkyl, Alkoxyalkyl, Cyanoalkyl, Alkylthioalkyl, Alkenyl, Halogenalkenyl, Alkinyl, Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy steht,
R¹ und R² unabhängig voneinander für Wasserstoff, Cyano, Alkyl, Halogenalkyl, Alkoxy, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, für gegebenenfalls substituierte Alkylcarbonyl, Alkoxycarbonyl, Arylcarbonyl, Hetarylcarbonyl, Aryloxycarbonyl, Hetaryloxycarbonyl, Alkylsulfinyl, Halogenalkylsulfinyl, Arylsulfinyl, Arylalkylsulfinyl, Hetarylsulfinyl, Hetarylalkylsulfinyl, Alkylsulfonyl, Halogenalkylsulfonyl, Arylsulfonyl, Arylalkylsulfonyl, Hetarylsulfonyl, Hetarylalkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen, wobei m für die Zahl 1, 2, 3 oder 4 steht, oder
R¹ und R² auch zusammen mit dem N-Atom, an das sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann, oder
R¹ und R² auch zusammen mit den Atomen, an die sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann,

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden definiert,
wobei
n bevorzugt für die Zahl 0, 1 oder 2 steht,
X¹, X², X³, X⁴ unabhängig von einander bevorzugt für Wasserstoff, Halogen, Hydroxy, Amino, OCN, SCN, SF₅, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₄)-alkoxy, (C₁-C₆)Alkoxycarbonyl-(C₁-C₄)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₇)Alkoxycarbonyl-(C₁-C₆)alkoxy, (C₁-C₆)Alkoxy-(C₁-C₆)alkoxy, (C₁-C₇)Alkylhydroxyimino, (C₁-C₇)Alkoxyimino, (C₁-C₆)Alkyl-(C₁-C₇)alkoxyimino, (C₁-C₆)Halogenalkyl-(C₁-C₇)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl,(C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C1-C₆)Alkylsulfonyloxy,(C₁-C₇)Alkylcarbonyl, (C₁-C₇)Halogenalkylcarbonyl, Carboxyl, (C₁-C₇)Alkylcarbonyloxy, (C₁-C₇)Alkoxycarbonyl, (C₁-C₇)Halogenalkoxycarbonyl, (C₁-C₇)Alkoxycarbonyl-(C₁-C₆)alkyl, Aminocarbonyl, (C₁-C₇)Alkylaminocarbonyl, Di-(C₁-C₇)alkylaminocarbonyl, (C₁-C₇)Alkenylaminocarbonyl, Di-(C₁-C₇)-alkenylaminocarbonyl, (C₃-C₈)Cycloalkylaminocarbonyl, (C₁-C₆)Alkylsulfonylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl,
oder für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl substituiertes gegebenenfalls durch ein oder zwei Heteroatome aus der Reihe O, S oder N unterbrochenes, gegebenenfalls gesättigtes oder ungesättigtes (C₃-C₈)Cycloalkyl, (C₃-C₈)Cycloalkoxy, (C₃-C₈)Cycloalkyl(C₁-C₆)alkyl oder (C₃-C₈)Cycloalkyl-(C₁-C₆)alkoxy substituiertes Phenyl-(C₁-C₆)alkyl, Phenoxy, Phenyl-(C₁-C₄)alkyloxy, Phenoxy-(C₁-C₄)alkyl, Phenylthio, Phenylthio-(C₁-C₄)alkyl, Phenylsulfinyl, Phenylsulfonyl, Hetaryl-(C₁-C₆)alkyl, Hetaryloxy, Hetaryl-(C₁-C₄)alkyloxy, Hetarylthio, Hetarylsulfinyl, Hetarylsulfonyl, gegebenenfalls gesättigtes oder ungesättigtes (C₃-C₈)Cycloalkyl-(C₁-C₆)alkyl, (C₃-C₈)Cycloalkyloxy, (C₃-C₈)Cycloalkyl-(C₁-C₆)alkoxy, (C₃-C₈)Cycloalkylthio, (C₃-C₈)Cycloalkyl-(C₁-C₆)alkylthio, (C₃-C₈)Cycloalkylsulfinyl, (C₃-C₈)Cycloalkyl-(C₁-C₆)alkylsulfinyl, (C₃-C₈)Cycloalkylsulfonyl, (C₃-C₈)Cycloalkyl-(C₁-C₆)alkylsulfonyl,
oder für NR⁴R⁵, wobei R⁴ und R⁵ unabhängig voneinander für Wasserstoff, Cyano, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkyl-(C₁-C₆)thioalkyl, (C₂-C₈)Alkenyl, (C₂-C₈)Halogenalkenyl, (C₂-C₈)Cyanoalkenyl, (C₂-C₈)Alkinyl, (C₂-C₈)Halogenalkinyl, (C₂-C₈)Cyanoalkinyl, Acyl, (C₁-C₇)Alkoxycarbonyl stehen oder R⁴ und R⁵ gemeinsam mit dem N-Atom, an dem sie gebunden sind, einen gegebenenfalls gleich oder verschieden durch Halogen, Cyano, Hydroxy, Amino, (C₁-C₆)Alkyl, (C₁-C₆)Halogenalkyl, (C₁-C₆)Alkoxy, (C₁-C₆)Halogenalkoxy, oder durch gegebenenfalls gleich oder verschieden durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl oder (C₃-C₈)Cycloalkyl(C₁-C₆)alkyl, substituierten und gegebenenfalls durch Heteroatome aus der Reihe O, S oder N unterbrochenen gesättigten oder ungesättigten fünf- bis siebengliedrigen Ring bilden können, stehen,
oder für einen (C₃-C₈)Cycloalkyl, (C₃-C₈)Cycloalkoxy, (C₃-C₈)Cycloalkylthio, (C₃-C₈)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden substituiert ist mit Halogen, Cyano, Nitro, (C₁-C₆)Alkyl, (C₁-C₆)Halogenalkyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, oder durch gegebenenfalls durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl(-C₁-C₆)alkyl oder (C₃-C₈)Cycloalkyl-(C₁-C₆)alkoxy steht,
oder, X² und X³, oder X³ und X⁴, können die folgenden 5 oder 6-gliedrigen Ring ausbilden, die gegebenenfalls gleich oder verschieden durch Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, subsitutiert sind, 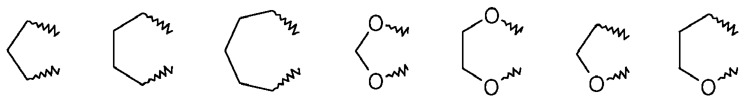 oder X² und X³, oder X³ und X⁴, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, (C₁-C₆)Alkoxy(C₁-C₆)alkyl, (C₁-C₆)Alkylthio, (C₁-C₆)Alkylsulfonyl, Amino, (C₁-C₆)Alkylamino, Di-(C₁-C₆)-alkylamino oder (C₃-C₈)Cycloalkylamino, 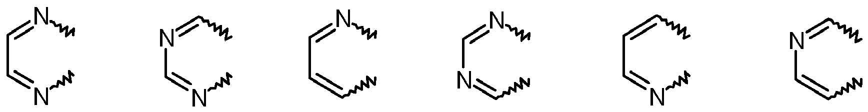 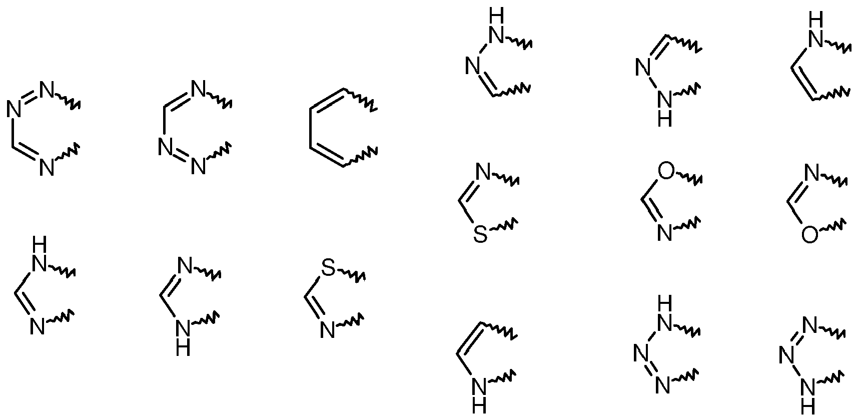 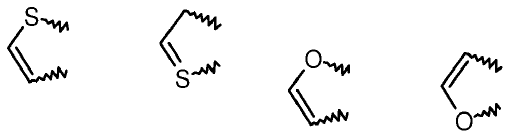 R³ bevorzugt für Wasserstoff, (C₂-C₆)Alkyl, Cyano, (C₁-C₆)Halogenalkyl, (C₁-C₆)Alkoxy-(C₁-C₆)alkyl, (C₁-C₆)Cyanoalkyl, (C₁-C₆)Alkylthio-(C₁-C₆)alkyl, (C₂-C₆)Alkenyl, (C₂-C₆)Halogenalkenyl, (C₂-C₆)Alkinyl, (C₂-C₆)Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₆)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder substituiert mit gegebenenfalls gleich oder verschieden durch Halogen, Cyano, Nitro, Hydroxy, Amino, Carboxy, (C₁-C₆)Alkyl, (C₁-C₆)Halogenalkyl, (C₁-C₆)Alkoxy, (C₁-C₆)Halogenalkoxy substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
R¹ und R² unabhängig voneinander bevorzugt für Wasserstoff, Cyano, (C₁-C₆)Alkyl, (C₁-C₆)Halogenalkyl, (C₁-C₆)Alkoxy, (C₁-C₆)Cyanoalkyl, (C₁-C₆)Hydroxyalkyl, (C₁-C₆)Alkoxy-(C₁-C₆)alkyl, (C₁-C₆)Alkylthio-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl,
für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylamino, Di-(C₁-C₆)alkyl-amino substituierte (C₁-C₆)Alkylcarbonyl, (C₁-C₇)Alkoxycarbonyl, Arylcarbonyl, Hetarylcarbonyl, Aryloxycarbonyl, Hetaryloxycarbonyl, (C₁-C₆)Alkylsulfinyl, (C₁-C₆)Halogenalkylsulfinyl, Arylsulfinyl, Aryl(C₁-C₆)alkylsulfinyl, Hetarylsulfinyl, Hetaryl(C₁-C₆)alkylsulfinyl, (C₁-C₆)Alkylsulfonyl, (C₁-C₆)Halogenalkylsulfonyl, Arylsulfonyl, Aryl(C₁-C₆)alkylsulfonyl, Hetarylsulfonyl, Hetaryl(C₁-C₆)alkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)-alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)-alkylaminosulfonyl, oder mit gegebenenfalls substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen,
oder für -(CH₂)ₘ₋R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkylamino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder gleich oder verschieden mit gegebenenfalls durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen, wobei m für die Zahl 1, 2 oder 3 steht, oder
R¹ und R² zusammen mit den Atomen, an das sie gebunden sind, einen gesättigten oder ungesättigten und gegebenenfalls einfach oder mehrfach durch (C₁-C₄)Alkyl, (C₁-C₄)Alkoxy, Halogen, (C₁-C₄)Halogenalkyl substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe stehen,
R¹ und R³ zusammen mit den Atomen, an die sie gebunden sind, einen gesättigten oder ungesättigten und gegebenenfalls einfach oder mehrfach gleich oder verschieden durch (C₁-C₄)Alkyl, (C₁-C₄)Alkoxy, Halogen, (C₁-C₄)Halogenalkyl, (C₂-C₄)Alkandiyl, (C₂-C₄)Alkendiyl, Butandienyl (wobei Alkandiyl, Alkendiyl und Butandienyl gegebenenfalls durch (C₁-C₄)Alkyl, (C₁-C₄)Alkoxy, Halogen, (C₁-C₄)Halogenalkyl substituiert sein können und/oder gegebenenfalls durch mindestens ein Sauerstoff- oder/und Stickstoffatom unterbrochen sein können) substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe enthalten kann,

Die erfindungsgemäßen Verbindungen sind durch die Formel (I) allgemein definiert. Besonders bevorzugte Substituenten bzw. Bereiche der in der oben und nachstehend erwähnten Formeln aufgeführten Reste werden definiert,
wobei
n besonders bevorzugt für die Zahl 0 oder 1 steht,
X¹, X², X³, X⁴ unabhängig von einander besonders bevorzugt für Wasserstoff, Fluor, Chlor, Brom, Jod, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, Aminothiocarbonyl,
oder für gegebenenfalls einfach oder zweifach gleich oder verschieden durch Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, oder durch gegebenenfalls durch ein Heteroatom aus der Reihe O, S oder N unterbrochenes, gegebenenfalls gesättigtes oder ungesättigtes (C₃-C₆)Cycloalkyl substituiertes Phenyl-(C₁-C₄)alkyl, Phenoxy, Hetaryl-(C₁-C₄)alkyl, Hetaryloxy, gegebenenfalls gesättigtes oder ungesättigtes (C₃-C₆)Cycloalkyl-(C₁-C₄)alkyl, (C₃-C₆)Cycloalkyloxy, (C₃-C₆)Cycloalkyl-(C₁-C₄)alkoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl, der gegebenenfalls einfach oder zweifach, gleich oder verschieden substituiert ist mit Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, (C₃-C₆)Cycloalkyl steht,
oder, X² und X³, oder X³ und X⁴, können die folgenden 5 oder 6-gliedrigen Ring ausbilden, die gegebenenfalls einfach bis vierfach durch Fluor oder (C₁-C₄)Alkyl subsitutiert sind, 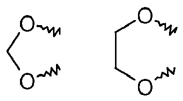 oder X² und X³, oder X³ und X⁴, können die folgenden anellierten Ringe ausbilden, die gegebenenfalls einfach oder zwei, gleich oder verschieden substituiert sind, wobei die Substituenten unabhängig voneinander ausgewählt sein können aus Wasserstoff, Fluor, Chlor, Brom, Cyano, (C₁-C₄)Alkyl, (C₃-C₆)Cycloalkyl, (C₁-C₄)Halogenalkyl, (C₁-C₄)Alkoxy, (C₁-C₄)Halogenalkoxy, 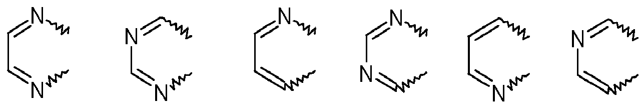 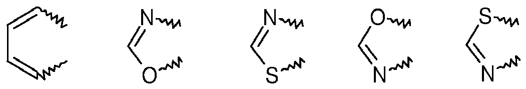 R³ besonders bevorzugt für Wasserstoff, (C₂-C₄)Alkyl, Cyano, (C₁-C₄)Halogenalkyl, (C₁-C₄)Alkoxy-(C₁-C₄)alkyl, (C₁-C₄)Cyanoalkyl,
oder für einen 3- bis 6-gliedrigen gesättigten, teilgesättigten oder aromatischen Ring, der gegebenenfalls ein bis zwei Heteroatome aus der Reihe O, S oder N enthalten kann und der gegebenenfalls einfach oder dreifach, gleich oder verschieden substituiert ist mit Fluor, Chlor, Brom, Jod, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, oder substituiert mit (C₃-C₆)Cycloalkyl steht,
R¹ und R² unabhängig voneinander besonders bevorzugt für Wasserstoff, Cyano, (C₁-C₄)Alkyl, (C₁-C₄)Halogenalkyl, (C₁-C₄)Alkoxy, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Alkinyl,
für gegebenenfalls einfach bis dreifach unabhängig voneinander durch Fluor, Chlor, Brom, Cyano, Nitro, (C₁-C₄)Alkyl, (C₁-C₄)Halogenalkyl, (C₂-C₄)Alkenyl, (C₂-C₄)Halogenalkenyl, (C₂-C₄)Alkinyl, (C₁-C₄)Alkoxy, (C₁-C₄)Halogenalkoxy, (C₁-C₄)Alkylthio, (C₁-C₄)Alkylsulfinyl, (C₁-C₄)Alkylsulfonyl, (C₁-C₄)Alkylamino, Di-(C₁-C₄)alkyl-amino substituiertes (C₁-C₄)Alkylcarbonyl, (C₁-C₅)Alkoxycarbonyl, Arylcarbonyl, Thiophenylcarbonyl, Pyridylcarbonyl, Pyrimidylcarbonyl, Thiazolylcarbonyl, Pyrazolylcarbonyl, (C₁-C₄)Alkylsulfinyl, (C₁-C₄)Halogenalkylsulfinyl, Arylsulfinyl, Aryl(C₁-C₄)alkylsulfinyl, Hetarylsulfinyl, Hetaryl(C₁-C₄)alkylsulfinyl, (C₁-C₄)Alkylsulfonyl, (C₁-C₄)Halogenalkylsulfonyl, Arylsulfonyl, Aryl(C₁-C₄)alkylsulfonyl, Hetarylsulfonyl, Hetaryl(C₁-C₄)alkylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen,
oder für -(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen, wobei m für die Zahl 1 oder 2 steht, oder
R¹ und R² zusammen mit dem N-Atom, an das sie gebunden sind, einen gesättigten oder ungesättigten und gegebenenfalls einfach oder vierfach durch Fluor, Chlor, (C₁-C₄)Alkyl, (C₁-C₄)Alkoxy, (C₁-C₄)Halogenalkyl substituierten 3- bis 6-gliedrigen Ring bilden können, der gegebenenfalls durch ein weiteres Heteroatom aus der Reihe Schwefel, Sauerstoff und Stickstoff enthalten kann und/oder mindestens eine Carbonylgruppe stehen,
R¹ und R³ zusammen mit den Atomen, an die sie gebunden sind, einen gesättigten oder ungesättigten und gegebenenfalls einfach oder mehrfach durch Methyl, Ethyl, Methoxy, Ethoxy, Fluor, Chlor, Trifluoromethyl, (C₂-C₄)Alkandiyl, (C₂-C₄)Alkendiyl, Butandienyl (wobei Butandienyl gegebenenfalls einfach oder zweifach durch Methyl, Fluor, Chlor, Brom, Methoxy oder Trifluoromethyl substituiert sein können und/oder gegebenenfalls durch mindestens ein Sauerstoff- oder/und Stickstoffatom unterbrochen sein können) substituierten 5- bis 6-gliedrigen Ring bilden können, der gegebenenfalls ein weiteres Schwefel- oder Sauerstoff- oder Stickstoffatom und/oder eine Carbonylgruppe enthalten kann,

Die erfindungsgemäßen Verbindungen sind durch die Formel (I) allgemein definiert. Ganz besonders bevorzugte Substituenten bzw. Bereiche der in der oben und nachstehend erwähnten Formeln aufgeführten Reste werden definiert,
wobei
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Difluoromethoxy, Trifluoromethoxy, OCH₂CF₃,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ ganz besonders bevorzugt für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl,
oder für Aryl steht, insbesondere bevorzugt für Phenyl steht,
R¹ ganz besonders bevorzugt für Wasserstoff, Methyl, Ethyl, Propyl, Butyl, *sec*-Butyl, *iso*-Propyl, *tert-*Butyl, (2,2,2)Trifluoroethyl, (2,2)Difluoromethyl, Methoxy, Ethoxy, Methoxymethyl, Methoxyethyl,
oder für Aryl steht, welches gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere wird Phenyl explizit genannt: welches gegebenenfalls mit den unter ganz besonders bevorzugt genannten Subtituenten substituiert sein kann,
oder für -(CH₂)ₘ-R⁶, wobei R⁶ für Aryl steht, welches gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere wird Phenyl für R⁶ explizit genannt, welches gegebenenfalls mit den unter ganz besonders bevorzugt genannten Subtituenten substituiert sein kann,
R² ganz besonders bevorzugt für Wasserstoff, Methyl oder Ethyl steht, oder
R¹ und R² zusammen mit dem N-Atom, an das sie gebunden sind, einen gesättigten oder ungesättigten 5- bis 6-gliedrigen Ring bilden können, der gegebenenfalls ein Sauerstoffatom enthalten kann, insbesondere werden folgende Ringe explizit genannt, 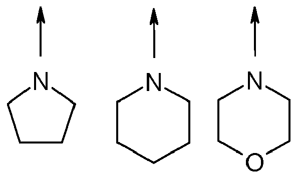 in denen das N-Atom, an das R¹ und R² gebunden sind, den Cyclus darstellen und der Pfeil zum Rest des Moleküls zeigt,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls eine Carbonylgruppe enthalten kann, insbesondere stehen R¹ und R³ zusammen mit den Atomen, an die sie gebunden sind für folgende Gruppe, 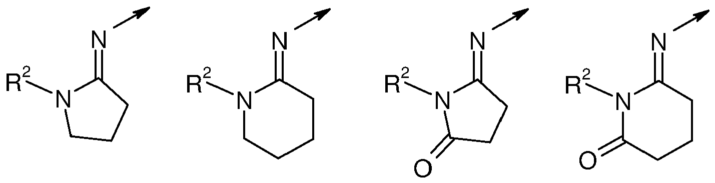 wobei der Pfeil zum Rest des Moleküls zeigt.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders bevorzugt stehen X² und X⁴ für folgende Kombinationen X²/X⁴: F/F, Cl/Cl, F/Cl, Cl/F, BrBr, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ ganz besonders bevorzugt für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ ganz besonders bevorzugt für
Cyano, Cyanomethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für Morpholin, Oxetan, Thienyl oder Pyridyl steht, die gegebenenfalls einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für Cyclopropyl, Cyclobutyl, Cyclopentyl, Cyclohexyl, steht, die einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für Aryl steht, insbesondere für Phenyl steht, welches einfach oder mehrfach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für -(CH₂)-R⁶, wobei R⁶ für Cyclopropyl oder Pyridyl steht, die gegebenenfalls einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder wobei R⁶ für Aryl steht, insbesondere für Phenyl steht, welches einfach oder mehrfach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der ein oder zwei weitere Heteroatome aus der Reihe Schwefel oder Stickstoff enthält,
oder zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 4-gliedrigen Ring bilden können, der gegebenenfalls ein weiteres Heteroatom aus der Reihe Sauerstoff, Schwefel oder Stickstoff enthält
insbesondere werden folgende Ringe explizit genannt, 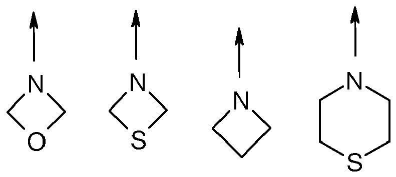 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
wobei
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ ganz besonders bevorzugt für Methoxymethyl, Methoxyethyl, Cyanomethyl, Allyl, Butenyl,
oder für Cyclopropyl, Pyridyl, Pyrimidyl, Thiazolyl, Pyrazolyl oder Thienyl steht, die gegebenenfalls einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert sein können,
oder für Aryl, insbesondere für Phenyl steht, welches einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert ist,
R¹ und R² ganz besonders bevorzugt unabhängig voneinander für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Morpholin, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 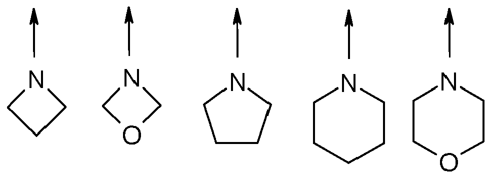 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Vinyl, Ethinyl, Methoxy, Ethoxy, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
R³ ganz besonders bevorzugt für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ und R² ganz besonders bevorzugt unabhängig voneinander für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Morpholin, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 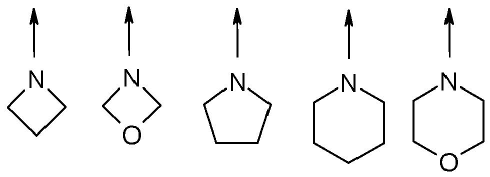 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 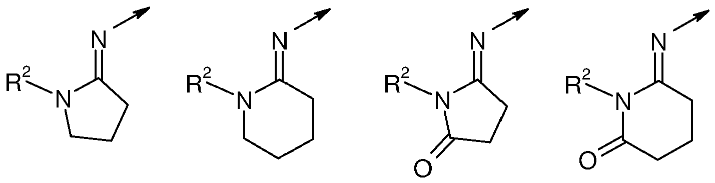 wobei der Pfeil zum Rest des Moleküls zeigt.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

In den als bevorzugt gennanten Restedefinitionen steht Halogen für Fluor, Chlor, Brom und Jod, besonders bevorzugt für Fluor, Chlor und Brom, und ganz besonders bevorzugt für Fluor und Chlor.

### Herstellverfahren

Die Verbindungen der allgemeinen Formel (I) können mit den in der Anmeldung WO 2007/131680 beschriebenen Methoden hergestellt werden. Abweichend von diesen beschriebenen Methoden können die Verbindungen der Formel (I) auch nach Verfahren A, Verfahren B oder Verfahren C hergestellt werden.

### Verfahren A

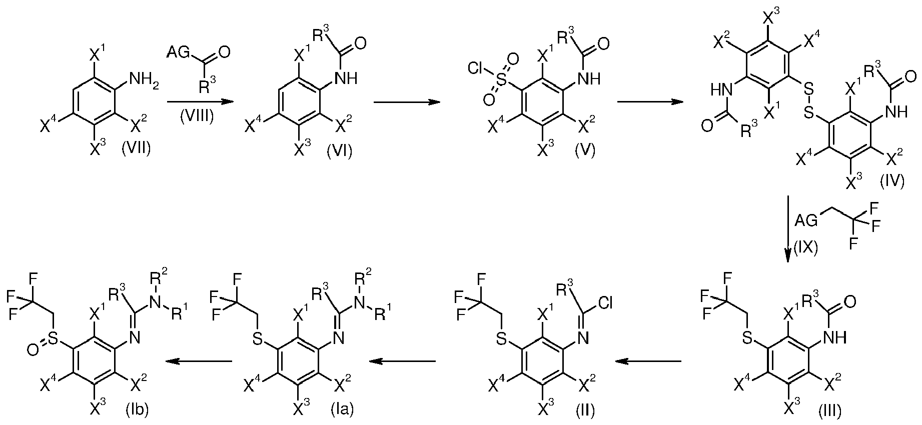

Wobei X¹, X², X³, X⁴, R¹, R² und R³ die oben angegebenen Bedeutungen haben, AG für eine Abgangsgruppe und PG für eine Schutzgruppe steht.

Aniline der Formel (VII) sind entweder kommerziell erhältlich oder können durch bekannte Methoden hergestellt werden. In Gegenwart von Säuren, Säureanhydriden oder Säurechloriden der Formel (VIII) werden die Aniline (VII) in die entsprechenden Anilide (VI) überführt. Die Chlorsulfonierung der Anilide (VI) mit Chlorsulfonsäure liefert die entsprechenden Sulfonylchloride (V). Die Reduktion der Sulfonylchloride (V) in die Disulfide (IV) ist mit literaturbekannten Methoden wie zum Beispiel Eisen in Salzsäure oder Iodid möglich. Die Umsetzung der Disulfide (IV) mit Trifluorethylelektrophilen der Formel (XVI), wobei AG für eine Abgangsgruppe wie zum Beispiel Chlor, Brom, Tosylat, Mesylat oder Triflat steht, liefern die Sulfide (III).

Die Anilide (III) werden beispielsweise mit Diphenylchlorphosphat oder Phosphorpentachlorid zu Imidoylchloriden der Formel (II) chloriert und mit Aminen zu Amidinen (Ia) umgesetzt.

Die Thioether (Ia) werden in die entsprechenden Sulfoxide (Ib) durch Umsetzung mit Oxidationsmitteln wie zum Beispiel meta-Chlorperbenzoesäure überführt.

Alternativ können die Thioether der Formel (III) nach Verfahren B hergestellt werden.

### Verfahren B

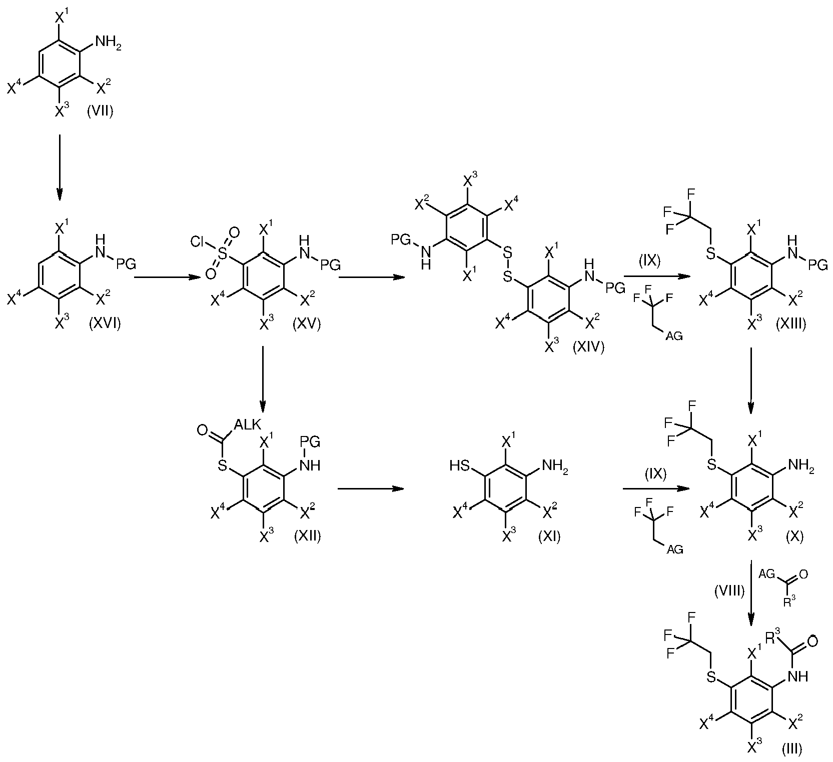

Die Aniline (VII) können mit einer geeigneten literaturbekannten Schutzgruppe wie zum Beispiel einer Acetylgruppe zu Verbindungen der Formel (XVI) geschützt werden. Die Chlorsulfonierung der Verbindungen der Formel (XVI) mit Chlorsulfonsäure liefert die entsprechenden Sulfonylchloride (XV). Die Reduktion der Sulfonylchloride (XV) in die Disulfide (XIV) ist mit literaturbekannten Methoden wie zum Beispiel Eisen in Salzsäure oder Iodid möglich. Die Umsetzung der Disulfide (XIV) mit Trifluorethylelektrophilen der Formel (IX), wobei AG für eine Abgangsgruppe wie zum Beispiel Chlor, Brom, Tosylat, Mesylat oder Triflat steht, liefern die Sulfide (XIII). Die Schutzgruppe wird durch geeignete literaturbekannte Methoden abgespalten, so dass man Aniline der Formel (X) erhält. Diese werden in Gegenwart von Säuren, Säureanhydriden oder Säurechloriden der Formel (VIII) in die entsprechenden Anilide (III) überführt.

Statt der Reduktion zum Disulfid (XIV), kann mit einem geeigneten Reduktionsmittel wie zum Beispiel Iod/Phosphor auch zum Alkylthioat (XI) reduziert und anschließend durch eine geeignete Methode wie zum Beispiel mit Kaliumhydoxidlösung zu Verbindungen der Formel (X) entschützt werden. Die Umsetzung der Thiole (XI) mit Trifluorethylelektrophilen der Formel (IX), wobei AG für eine Abgangsgruppe wie zum Beispiel Chlor, Brom, Tosylat, Mesylat oder Triflat steht, liefern die Sulfide (X). Diese werden in Gegenwart von Säuren, Säureanhydriden oder Säurechloriden der Formel (VIII) in die entsprechenden Anilide (III) überführt.

### Verfahren C

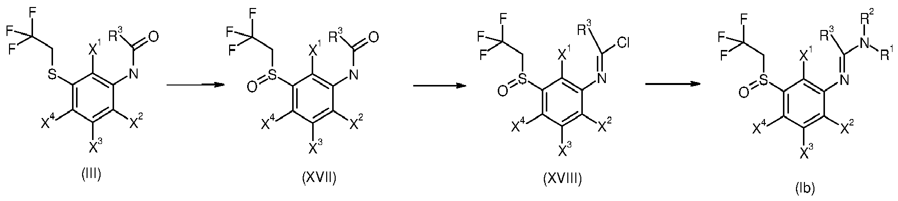

Weiterhin wurde gefunden, dass man chirale Verbindungen (Ib) nach Verfahren C erhält, wenn man Verbindungen der Formel (III) chiral oxidiert zu Sulfoxide der Formel (XVII).

Die Anilide (XVII) werden beispielsweise mit Diphenylchlorphosphat oder Phosphorpentachlorid zu Imidoylchloriden der Formel (XVIII) chloriert und mit Aminen zu Amidinen (Ib) umgesetzt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Diplopoda z.B. Blaniulus guttulatus.
Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..
Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphorenthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z. B. hohen oder niedrigen Temperaturen, gegen Trockenheit oder gegen erhöhten Wasser- bzw. Bodensalzgehalt. Auch lässt sich das Blüh- und Fruchtverhalten verbessern, die Keimfähigkeit und Bewurzelung optimieren die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die folgenden:

### Insektizide/Akarizide/Nematizide

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

### (1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder

Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.

### (2) GABA-gesteuerte Chlorid-Kanal-Antagonisten, wie beispielsweise

Cyclodien-organochlorine, z.B. Chlordane und Endosulfan; oder
Phenylpyrazole (Fiprole), z.B. Ethiprole und Fipronil.

### (3) Natrium-Kanal-Modulatoren / Spannungsabhängige Natrium-Kanal-Blocker, wie beispielsweise

Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(IR)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
DDT; oder Methoxychlor.

### (4) Nikotinerge Acetylcholin-Rezeptor (nAChR) Agonisten, wie beispielsweise

Neonikotinoide, z.B. Acetamiprid, Clothianidin, Dinotefuran, Imidacloprid, Nitenpyram, Thiacloprid und Thiamethoxam; oder
Nikotin.

### (5) Nikotinerge Acetylcholin-Rezeptor (nAChR) allosterische Aktivatoren, wie beispielsweise

Spinosine, z.B. Spinetoram und Spinosad.

### (6) Chlorid-Kanal-Aktivatoren, wie beispielsweise

Avermectine/Milbemycine, z.B. Abamectin, Emamectin-benzoat, Lepimectin und Milbemectin.

### (7) Juvenilhormon-Imitatoren, wie beispielsweise

Juvenilhormon-Analoge, z.B. Hydroprene, Kinoprene und Methoprene; oder
Fenoxycarb; oder Pyriproxyfen.

### (8) Wirkstoffe mit unbekannten oder nicht spezifischen Wirkmechanismen, wie beispielsweise

Alkylhalide, z.B. Methylbromid und andere Alkylhalide; oder
Chloropicrin; oder Sulfurylfluorid; oder Borax; oder Brechweinstein.

### (9) Selektive Fraßhemmer, z.B. Pymetrozine; oder Flonicamid.

### (10) Milbenwachstumsinhibitoren, z.B. Clofentezine, Hexythiazox und Diflovidazin; oder Etoxazole.

### (11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, Cry1Fa, Cry2Ab, mCty3A, Cry3Ab, Cry3Bb, Cry34/35Ab1.

### (12) Inhibitoren der oxidativen Phosphorylierung, ATP-Disruptoren, wie beispielsweise Diafenthiuron; oder Organozinnverbindungen, z.B. Azocyclotin, Cyhexatin und Fenbutatin-oxid; oder Propargite; oder Tetradifon.

### (13) Entkoppler der oxidativen Phoshorylierung durch Unterbrechung des H-Protongradienten, wie beispielsweise Chlorfenapyr, DNOC und Sulfluramid.

### (14) Nikotinerge Acetylcholin-Rezeptor-Antagonisten, wie beispielsweise Bensultap, Cartap-hydrochlorid, Thiocyclam und Thiosultap-Natrium.

### (15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

### (16) Inhibitoren der Chitinbiosynthese, Typ 1, wie beispielsweise Buprofezin.

### (17) Häutungsstörende Wirkstoffe, Dipteran, wie beispielsweise Cyromazine.

### (18) Ecdyson-Rezeptor Agonisten, wie beispielsweise Chromafenozide, Halofenozide, Methoxyfenozide und Tebufenozide.

### (19) Oktopaminerge Agonisten, wie beispielsweise Amitraz.

### (20) Komplex-III-Elektronentransportinhibitoren, wie beispielsweise Hydramethylnon; oder Acequinocyl; oder Fluacrypyrim.

### (21) Komplex-I-Elektronentransportinhibitoren, beispielsweise

METI-Akarizide, z.B. Fenazaquin, Fenpyroximate, Pyrimidifen, Pyridaben, Tebufenpyrad und Tolfenpyrad; oder
Rotenone (Derris).

### (22) Spannungsabhängige Natriumkanal-Blocker, z.B. Indoxacarb; oder Metaflumizone.

### (23) Inhibitoren der Acetyl-CoA-Carboxylase, wie beispielsweise

Tetron- und Tetramsäurederivate, z.B. Spirodiclofen, Spiromesifen und Spirotetramat.

### (24) Komplex-IV-Elektronentransportinhibitoren, wie beispielsweise

Phosphine, z.B. Aluminiumphosphid, Calciumphosphid, Phosphin und Zinkphosphid; oder
Cyanid.

### (25) Komplex-II-Elektronentransportinhibitoren, wie beispielsweise Cyenopyrafen.

### (28) Ryanodinrezeptor-Effektoren, wie beispielsweise

Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus W02005/077934), 4-{[(6-Brompyrid-3-yl)methyl](2-fluorethyl)amino}furan-2(SH)-on (bekannt aus W02007/115644), 4-{[(6-Fluorpyrid-3-yl)methyl](2,2-difluorethyl)amino}furan-2(5H)-on (bekannt aus W02007/115644), 4-{[(2-Chlor-1,3-thiazol-5-yl)methyl](2-fluorethyl)amino}furan-2(5H)-on (bekannt aus W02007/115644), 4-{[(6-Chlorpyrid-3-yl)methyl](2-fluorethyl)amino}furan-2(5H)-on (bekannt aus W02007/115644), Flupyradifurone, 4-{[(6-Chlor-5-fluorpyrid-3-yl)methyl](methyl)amino}furan-2(5H)-on (bekannt aus W02007/115643), 4-{[(5,6-Dichlorpyrid-3-yl)methyl](2-fluorethyl)amino}furan-2(5H)-on (bekannt aus W02007/115646), 4-{[(6-Chlor-5-fluorpyrid-3-yl)methyl](cyclopropyl)amino}furan-2(5H)-on (bekannt aus W02007/115643), 4-{[(6-Chlorpyrid-3-yl)methyl](cyclopropyl)amino}furan-2(5H)-on (bekannt aus EP-A-0 539588), 4-{[(6-Chlorpyrid-3-yl)methyl](methyl)amino}furan-2(5H)-on (bekannt aus EP-A-0539588), {[1-(6-Chlorpyridin-3-yl)ethyl](methyl)oxido-λ⁴-sulfanyliden}cyanamid (bekannt aus W02007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus W02007/149134) sowie Sulfoxaflor und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus W02006/089633), 3-(4'-Fluor-2,4-dimethylbiphenyl-3-yl)-4-hydroxy-8-oxa-1-azaspiro[4.5]dec-3-en-2-on (bekannt aus W02008/067911), 1-{2-Fluor-4-methyl-5-[(2,2,2-trifluorethyl)sulfinyl]phenyl}-3-(trifluormethyl)-1H-1,2,4-triazol-5-amin (bekannt aus W02006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus W02008/066153), 2-Cyan-3-(difluormethoxy)-N,N-dimethylbenzolsulfonamid (bekannt ausW02006/056433), 2-Cyan-3-(difluormethoxy)-N-methylbenzolsulfonamid (bekannt aus W02006/100288), 2-Cyan-3-(difluormethoxy)-N-ethylbenzolsulfonamid (bekannt aus W02005/035486), 4-(Difluormethoxy)-N-ethyl-N-methyl-1,2-benzothiazol-3-amin-1,1-dioxid (bekannt aus W02007/057407), N-[1-(2,3-Dimethylphenyl)-2-(3,5-dimethylphenyl)ethyl]-4,5-dihydro-1,3-thiazo1-2-amin (bekannt aus W02008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus W02003/106457), 3-(2,5-Dimethylphenyl)-4-hydroxy-8-methoxy-1,8-diazaspiro[4.5]dec-3-en-2-on (bekannt aus W02009/049851), 3-(2,5-Dimethylphenyl)-8-methoxy-2-oxo-1,8-diazaspiro[4.5]dec-3-en-4-yl-ethylcarbonat (bekannt aus W02009/049851), 4-(But-2-in-1-yloxy)-6-(3,5-dimethylpiperidin-1-yl)-5-fluorpyrimidin (bekannt aus W02004/099160), (2,2,3,3,4,4,5,5-Octafluorpentyl)(3,3,3-trifluorpropyl)malononitril (bekannt aus W02005/063094), (2,2,3,3,4,4,5,5-Octafluorpentyl)(3,3,4,4,4-pentafluorbutyl)malononitril (bekannt aus W02005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus W02007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus W02007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus W02007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus W02005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus W02010/005692), NNI-0711 (bekannt aus W02002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus W02002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus W02005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus W02005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus W02005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus W02005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus W02005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus W02007/101369), 2-{6-[2-(5-Fluorpyridin-3-yl)-1,3-thiazol-5-yl]pyridin-2-yl}pyrimidin (bekannt aus W02010/006713), 2-{6-[2-(Pyridin-3-yl)-1,3-thiazol-5-yl]pyridin-2-yl}pyrimidin (bekannt aus W02010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus W02010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus W02010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus W02010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus W02010/069502), (lE)-N-[(6-Chlorpyridin-3-yl)methyl]-N'-cyan-N-(2,2-difluorethyl)ethanimidamid (bekannt aus W02008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus W02011/049233).

### Fungizide

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).
(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus W02010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).
(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).
(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).
(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).
(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus W02005070917).
(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).
(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).
(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).
(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus W02005042474).
(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).
(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).
(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).
(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus W02003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus W02005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus W02009094442), (15.75) 5-Fluor-2-[(4-methylbenzyl)oxy]pyrimidin-4-amin (1174376-25-0) (bekannt aus W02009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{ [5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxanüd, (16.3) N-(2',4'-Dichlorbiphenyl-2-yl)-3-(difluormethyl)-1-methyl-1H-pyrazol-4-carboxanüd, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yll-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken.

### Herstellbeispiel 1: 2,2,2-Trifluor-N'-{2-fluor-4-methyl-5-[(2,2,2-trifluorethyl)sulfinyl]phenyl}-N,N-dimethylethanimidamid (Ib-01)

### Stufe 1: 2,2,2-Trifluor-N-(2-fluor-4-methylphenyl)acetamid (VI-1)

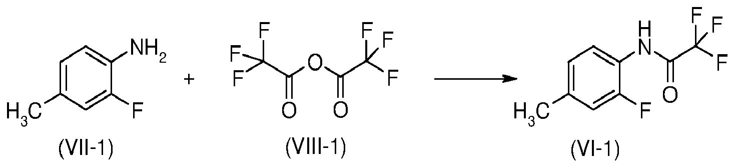

27,5 g 2-Fluor-4-Methylanilin werden bei 0 °C in 300 mL Dichlormethan vorgelegt, 26,7 g Triethylamin werden zugegeben und anschließend werden 50,8 g Trifluoressigsäureanhydrid zugetropft. Es wird 2 h bei 0 °C nachgerührt und anschließend einrotiert. Der Rückstand wird in Wasser aufgenommen und zweimal mit Ethylacetat extrahiert. Die vereinigten organischen Phasen werden über Natriumsulfat getrocknet, filtriert, und das Lösemittel wird im Vakuum entfernt. Es werden 49,0 g (100% d.Th.) des Trifluoracetamids (VI-1) erhalten.

### logP(HCOOH): 2,40

### Stufe 2: 4-Fluor-2-methyl-5-[(trifluoracetyl)amino]benzolsulfonylchlorid (V-1)

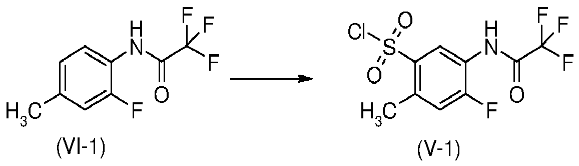

258 g Chlorsulfonsäure werden vorgelegt und bei Raumtemperatur werden 49 g 2,2,2-Trifluor-N-(2-fluor-4-methylphenyl)acetamid (VI-1) in Portionen zugesetzt. Bei Raumtemperatur wird noch 16 h nachgerührt. Die Mischung wird unter Rühren auf Eis gegeben und zweimal mit Ethylacetat extrahiert. Die vereinigten organischen Phasen werden über Natriumsulfat getrocknet, filtriert, und das Lösemittel wird im Vakuum entfernt. Es werden 70,8 g des Chlorsulfonyls (V-1) erhalten. Das Rohprodukt wird sofort weiter umgesetzt.

### Stufe 3: N,N'-[Disulfanediylbis(6-fluor-4-methylbenzol-3,1-diyl)]bis(2,2,2-trifluoracetamid) (IV-1)

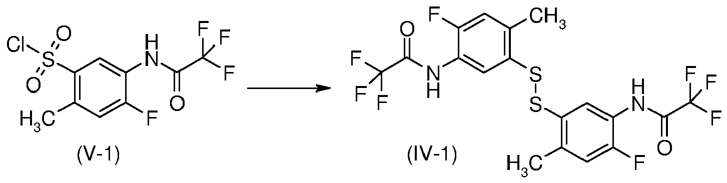

298,8 g Natriumiodid werden in 1000 mL Trifluoressigsäure gelöst und 70,8 g 4-Fluor-2-methyl-5-[(trifluoracetyl)amino]benzolsulfonylchlorid (V-1) werden bei Raumtemperatur zugegeben. Es wird 16 h bei Raumtemperatur nachgerührt und anschließend das Lösemittel im Vakuum entfernt. Der Rückstand wird mit Wasser verrührt und abgesaugt. Es werden 62,3 g (86% d.Th.) des Disufids (IV-1) als Feststoff erhalten.

### logP(HCOOH): 4,41

### Stufe 4: 2,2,2-Trifluor-N-{2-fluor-4-methyl-5-[(2,2,2-trifluorethyl)sulfanyl]phenyl}acetamid (III-1)

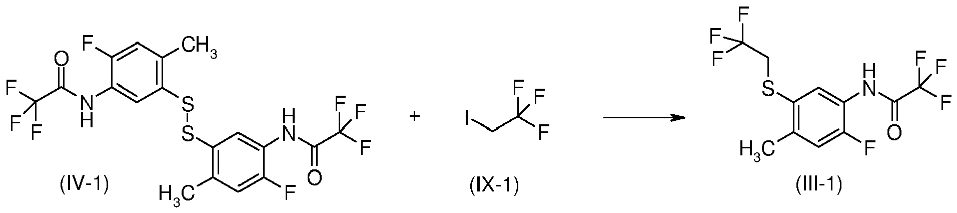

### 3,4 g N,N'-[Disulfanediylbis(6-fluor-4-methylbenzol-3,1-diyl)]bis(2,2,2-trifluoracetamid) (IV-1) werden in 150 ml N,N-Dimethylformamid gelöst und mit 1,86 g Kaliumcarbonat, 3,11 g 1,1,1-Trifluoriodethan (XVI-1), 2,39 g Rongalit und einigen Tropfen Wasser versetzt. Die Reaktionsmischung wird 16 h bei Raumtemperatur gerührt. Die Hauptmenge des N,N-Dimethylformamids wird im Vakuum abdestilliert. Der Rückstand wird in Wasser aufgenommen und zweimal mit Ethylacetat extrahiert. Die vereinigten organischen Phasen werden über Natriumsulfat getrocknet, filtriert, und anschließend wird das Lösemittel im Vakuum entfernt. Es werden 4,48 g (90% d.Th.) des Thioethers (III-1) erhalten.

### logP(HCOOH): 3,31

### Stufe 5: 2,2,2-Trifluor-N-{2-fluor-4-methyl-5-[(2,2,2-trifluorethyl)sulfanyl]phenyl} ethanimidoylchlorid (II-1)

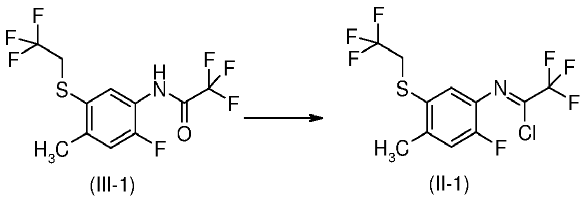

1 g 2,2,2-Trifluor-N-{2-fluor-4-methyl-5-[(2,2,2-trifluorethyl)sulfanyl]phenyl}acetamid (III-1), 1,51 g Triethylamin und 4,01 g Diphenylchlorphosphat werden in 20 mL Acetonitril 16 h refluxiert. Nach Abkühlen wird Ethylacetat zugegeben, der ausgefallene Feststoff abfiltriert und verworfen. Das Filtrat wird auf Kieselgel gezogen und mit Cyclohexan/Ethylacetat (98/2) chromatographiert. Nach Entfernen des Lösemittels im Vakuum erhält man 1 g des Imidoylchlorids (II-1).

### Stufe 6: 2,2,2-Trifluor-N'-{2-fluor-4-methyl-5-[(2,2,2-tlifluorethyl)sulfanyl]phenyl -N,N-dimethylethanimidamid (Ia-01)

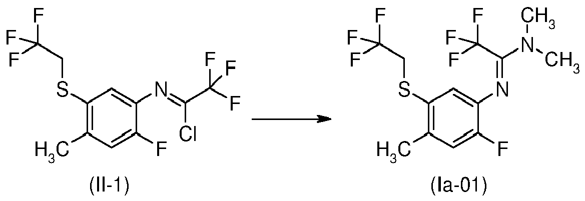

0,77 g Dimethylamin (2 M in THF) werden in 40 ml Acetonitril vorgelegt, und bei Raumtemperatur wird 1 g 2,2,2-Trifluor-N-{2-fluor-4-methyl-5-[(2,2,2-trifluorethyl)sulfanyl]phenyl} ethanimidoylchlorid (II-1) gelöst in 10 mL Acetonitril zugetropft. Die Reaktionsmischung wird 16 h bei Raumtemperatur gerührt und anschließend wird das Lösemittel im Vakuum entfernt. Der Rückstand wird in Wasser aufgenommen und zweimal mit Ethylacetat extrahiert. Die vereinigten organischen Phasen werden über Natriumsulfat getrocknet, filtriert und anschließend wird das Lösemittel im Vakuum entfernt. Es werden 0,36 g (35% d.Th.) des Amidins (Ia-01) erhalten.

### logP(HCOOH): 4,48

### Stufe 7: 2,2,2-Trifluor-N'-{2-fluor-4-methyl-5-[(2,2,2-trifluorethyl)sulfinyl]phenyl}-N,N-dimethylethanimidamid (Ib-01)

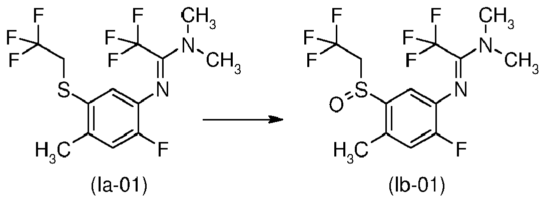

0,36 g 2,2,2-Trifluor-N'-{2-fluor-4-methyl-5-[(2,2,2-trifluorethyl)sulfanyl]phenyl}-N,N-dimethylethanimidamid (Ia-01) werden in 30 mL Dichlormethan gelöst und bei Raumtemperatur werden 0,21 g meta-Chlorperbenzoesäure zugegeben. Die Reaktionsmischung wird 16 h bei Raumtemperatur nachgerührt und anschließend mit Natriumcarbonatlösung alkalisch gestellt. Überschüssige meta-Chlorperbenzoesäure wird mit Natriumthiosulfat reduziert. Nach Phasentrennung wird das Lösemittel im Vakuum entfernt. Der Rückstand wird mit Cyclohexan/Aceton (9/1) chromatographiert. 0,31 g (79% d.Th) des Amidins (Ib-01) werden so erhalten.

### logP(HCOOH): 3,15

### Herstellbeispiel 2: 5-Amino-4-fluor-2-methylbenzolthiol (XI-1)

### Stufe 1: S-(5-Acetamido-4-fluor-2-methylphenyl)ethanthioat (XII-1)

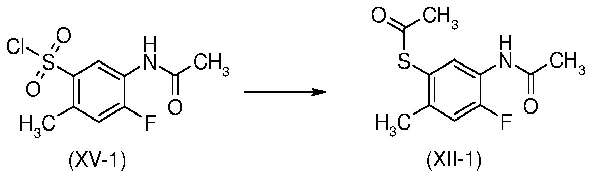

99,3 g 5-Acetamido-4-fluor-2-methylbenzolsulfonylchlorid (XV-1) werden in 700 mL Eisessig suspendiert, mit 0,9 g Iod und 38,7 g rotem Phosphor versetzt und 5 h bei Rückfluß nachgerührt. Nach Abkühlen wird der Feststoff abfiltriert und das Filtrat einrotiert. Der Rückstand wird mit Wasser verrührt und abgesaugt. 57,6 g (67% d.Th.) des Thioats (XII-1) werden als Feststoff erhalten.

### logP(HCOOH): 1,78

### Stufe 2: 5-Amino-4-fluor-2-methylbenzolthiol (XI-1)

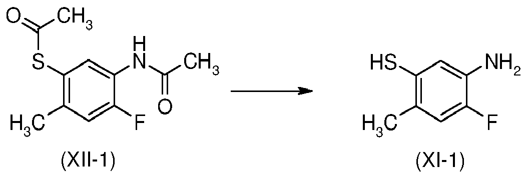

57,4 g S-(5-Acetamido-4-fluor-2-methylphenyl)ethanthioat (XII-1) werden in 750 mL Wasser und 96,6 g Kaluimhydroxid gelöst. Die Reaktionsmischung wird 16 h bei Rückfluß gekocht. Nach Abkühlen wird die Lösung mit Salzsäure auf pH 2-3 gestellt und der ausgefallene Feststoff abgesaugt. 35,8 g (94% d.Th.) des Thiols (XI-1) werden als Feststoff erhalten.

### logP(HCOOH): 3,70

### Chirale Oxidation von (III) zu (XVII)

### Beispiel 1: Synthese von 2,2,2-Trifluor-N-{4-fluor-2-methyl-5-[(2,2,2-trifluorethyl)sulfinyl]-phenyl}acetamid

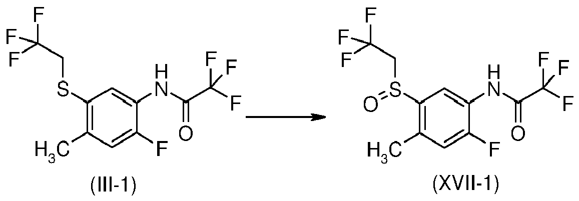

In einem Dreihalskolben wurden 500 mg (1,49 mmol) 2,2,2-Trifluor-N-{4-fluor-2-methyl-5-[(2,2,2-trifluorethyl)sulfanyl]phenyl}acetamid in 5 g Chloroform gelöst und auf 15 °C abgekühlt. Zu dieser Mischung wurde eine Lösung aus 15,82 mg (0,06 mmol) Vanadiumacetylacetonat und 29,84 mg (0,089 mmol) (S)(2,4-Di-*tert*.-butyl-6-{(E)-[(1-hydroxy-3,3-dimethylbutan-2-yl)imino]methyl}phenol 1 g Chloroform zugesetzt. Nach 5 Minuten wurde eine Lösung aus 225,5 mg (1,79 mmol) 30%iger H₂O₂ und 300 mg Puffer-Lösung pH 7 (KH₂PO₄/Na₂HPO₄) über 20 Minuten zudosiert. Der Verlauf der Umsetzung wurde mittels HPLC verfolgt. Nach 2 h Reaktionszeit wurden 100 mg Thiosulfatlösung zugesetzt und Chloroform weitgehend im Vakuum verdampft. Zu dem Rückstand wurden 5 g Cyclohexan zugegeben und der ausgefallene Feststoff wurde abfiltriert.

Man erhielt 1-{2,4-Dimethyl-5-[(2,2,2-trifluorethyl)-sulfinyl]phenyl}-3-(trifluormethyl)-1H-1,2,4-triazol (91,24% HPLC-Reinheit) als beigen Feststoff. Der Enantiomerenüberschuss wurde mittels HPLC an chiraler Phase (Daicel Chiracel OJ-RH 150) mit einem Verhältnis von 25,90 : 74,10 bestimmt.

Gemäß den oben beschriebenen Herstellverfahren lassen sich die Verbindungen der Formel (I) erhalten, beispielsweise die folgenden Verbindungen der Formel (I):

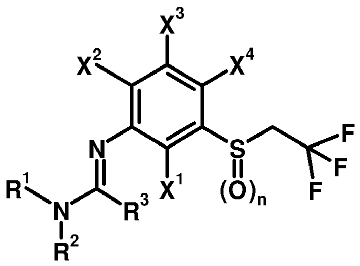

| **Bsp** | **n** | **R¹** | **R²** | **R³** | **X ¹** | **X²** | **X³** | **X⁴** | **Log P** |
|---|---|---|---|---|---|---|---|---|---|
| Ia-01 | 0 | CH₃ | CH₃ | CF₃ | H | F | H | CH₃ | 4,48^{[a]};4,5^{[b]} |
| Ia-02 | 0 | Propyl | H | CF₃ | H | F | H | CH₃ | 4,45^{[a]};4,43^{[b]} |
| Ia-03 | 0 | H | H | CHF₂ | H | F | H | CH₃ | 2,2^{[a]} |
| Ia-04 | 0 | Pyridin-2-ylmethyl | H | CF₃ | H | F | H | CH₃ | 3,68^{[a]} |
| Ia-05 | 0 | Propan-2-yl | H | CF₃ | H | F | H | CH₃ | 4,51^{[a]};4,52^{[b]} |
| Ia-06 | 0 | H | H | PD-F-Heptyl | H | F | H | CH₃ | |
| Ia-07 | 0 | H | H | CF₃ | H | CH₃ | H | CH₃ | 3,36^{[a]};3,4^{[b]} |
| Ia-08 | 0 | Pyridin-2-ylmethyl | Pyridin-2-ylmethyl | CF₃ | H | F | H | CH₃ | |
| Ia-09 | 0 | CH₃ | H | CF₃ | H | CH₃ | H | F | 3,72^{[a]};3,69^{[b]} |
| Ia-10 | 0 | CH₃ | CH₃ | DD-F-Hexyl | H | F | H | CH₃ | |
| Ia-11 | 0 | Methylsulfonyl | H | CF₃ | H | F | H | CH₃ | 3,05^{[a]} |
| Ia-12 | 0 | CH₃ | H | CF₃ | H | CH₃ | H | CH₃ | 3,95^{[a]};4,01^{[b]} |
| Ia-13 | 0 | CH₃ | H | CF₃ | H | F | H | CH₃ | 3,7^{[a]};3,71^{[b]} |
| Ia-14 | 0 | H | H | CF₃ | H | Cl | H | Cl | |
| Ia-15 | 0 | CH₃ | H | CF₃ | H | Cl | H | Cl | 4,2^{[a]};4,2^{[b]} |
| Ia-16 | 0 | (Trifluormethyl)sulf onyl | H | CF₃ | H | F | H | CH₃ | |
| Ia-17 | 0 | H | H | CF₃ | H | CH₃ | H | F | 3,16^{[a]};3,14^{[b]} |
| Ia-18 | 0 | Ethyl | H | CF₃ | H | F | H | CH₃ | 4,12^{[a]};4,12^{[b]} |
| Ia-19 | 0 | CH₃ | H | CF₃ | H | H | H | CF₃ | |
| Ia-20 | 0 | CH₃ | H | CF₃ | H | Cl | H | CH₃ | |
| Ia-21 | 0 | H | H | CF₃ | H | F | H | CH₃ | 3,13^{[a]};3,17^{[a]};3, 12^{[b]} |
| Ia-22 | 0 | H | H | DD-F-Hexyl | H | F | H | CH₃ | |
| Ia-23 | 0 | CH₃ | CH₃ | CF₃ | H | CH₃ | H | CH₃ | 4,87^{[a]};4,87^{[b]} |
| Ia-24 | 0 | CH₃ | CH₃ | CF₃ | H | CH₃ | H | F | 4,53^{[a]};4,47^{[b]} |
| Ia-25 | 0 | H | H | CF₃ | H | H | H | CF₃ | |
| Ia-26 | 0 | Cpr | H | CF₃ | H | F | H | CH₃ | 4,03^{[a]};4,02^{[b]} |
| Ib-01 | 1 | CH₃ | CH₃ | CF₃ | H | F | H | CH₃ | |
| Ib-02 | 1 | Ethyl | H | CF₃ | H | F | H | CH₃ | |
| Ib-03 | 1 | Propan-2-yl | | H | C F₃ | H | F | H | CH₃ |
| Ib-04 | 1 | CH₃ | H | CF₃ | H | CH₃ | H | CH₃ | 3,15^{[a]};3,09^{[b]} |
| Ib-05 | 1 | CH₃ | H | CF₃ | H | CH₃ | H | F | 2,86^{[a]};2,79^{[b]} |
| Ib-06 | 1 | Propyl | H | CF₃ | H | F | H | CH₃ | 3,24^{[a]};3,17^{[b]} |
| Ib-07 | 1 | CH₃ | H | CF₃ | H | F | H | CH₃ | 2,64^{[a]};2,61^{[b]} |
| Ib-08 | 1 | Cpr | H | CF₃ | H | F | H | CH₃ | 2,68^{[a]};2,67^{[b]} |
| Ib-09 | 1 | CH₃ | CH₃ | CF₃ | H | CH₃ | H | CH₃ | 3,23^{[a]};3,19^{[b]} |
| Ib-10 | 1 | CH₃ | H | CF₃ | H | H | H | CF₃ | 2,52^{[a]};2,49^{[b]} |
| Ib-11 | 1 | H | H | CF₃ | H | F | H | CH₃ | 2,77^{[a]};2,71^{[b]} |
| Ib-12 | 1 | H | H | CF₃ | H | CH₃ | H | CH₃ | 3,36^{[a]};3,3^{[b]} |
| Ib-13 | 1 | H | H | CF₃ | H | CH₃ | H | F | |
| Ib-14 | 1 | H | H | CF₃ | H | Cl | H | Cl | 2,05^{[a]};2,02^{[b]} |
| Ib-15 | 1 | CH₃ | CH₃ | CF₃ | H | CH₃ | H | F | 2,17^{[a]};2,14^{[b]} |
| Ib-16 | 1 | H | H | CF₃ | H | H | H | CF₃ | 2,2^{[a]};2,19^{[b]} |

| | | | | | | | | | |
|---|---|---|---|---|---|---|---|---|---|
| **Abkürzungen:** PD-F-Heptyl = Pentadecafluorheptyl;DD-F-Hexyl = 1,1,2,2,3,3,4,4,5,5,6,6-Dodecafluorhexyl;CHF₂ = Difluormethyl;Cpr = Cyclopropyl;Cl = Chlor;F = Fluor. Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden: ^{[a]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. ^{[b]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. ^{[c]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril. | | | | | | | | | |

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

Die NMR-Daten ausgewählter Beispiele werden entweder in klassischer Form (δ-Werte, Anzahl der H-Atome, Multiplettaufspaltung) oder als NMR-Peak-Listen aufgeführt.

### NMR-Peak-Listenverfahren

Wenn die 1H-NMR-Daten ausgewählter Beispiele in Form von 1H-NMR-Peaklisten notiert werden, wird zu jedem Signalpeak erst der δ-Wert in ppm und dann die Signalintensität durch ein Leerzeichen getrennt aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ Intensität₁;δ₂ Intensität₂;........;δᵢ Intensitätᵢ;..... ;δₙ Intensitätₙ

Das Lösungsmittel, in welchem das NMR-Spektrum aufgenommen wurde, wird in eckigen Klammern hinter der Nummer des Beispieles und vor der NMR-Peakliste bzw. der klassischen NMR-Interpretationsliste aufgeführt.

| **Beispielsnummer** | **1H-NMR Daten** |
|---|---|
| Ia-01 | 1H-NMR(D6-DMSO): 7,12(d,1H), 7,07(d,1H), 3,84-3,92(m,2H), 2,98(breit,6H), 2,33(s,3H) |
| Ia-02 | 1H-NMR (CDCl3): 7,03-7,09(m,1H), 6,91-6,93(m,1H), 5,29(breit,1H), 3,28-3,38 (m,4H), 2,42(s,3H), 1,69 (breit, 2H), 1,01(breit,2H), 0,83-0,88(m,1H) |
| Ia-03 | 1H-NMR(D6-DMSO): 7,11-7,14(m,1H), 7,06-7,04(m,1H), 6,92-6,98(breit,2H), 6,30(tt,1H), 3,83-3,91(q,2H), 2,34(s,3H) |
| Ia-05 | [CDCl₃] 7.26 45.31;7.09 0.35;7.03 0.72;6.93 0.95;6.91 0.85;5.09 0.50;4.13 0.50;3.30 1.93;3.28 1.82;2.42 16.00;1.57 44.22;1.43 0.36;1.33 0.54;1.29 4.62;1.28 4.60;1.26 1.33;1.25 1.06;1.25 1.27;1.23 0.74;1.08 0.47;0.01 0.72;0.00 24.21;-0.01 0.77 |
| Ia-07 | 1H-NMR(D6-DMSO): 7,10(s,1H), 6,98(breit, 2H), 6,85(s,1H), 3,87(q,2H), 2,30(s,3H), 1,98(s,3H) |
| Ia-09 | [DMSO-D₆] 7.09 3.30;7.07 3.26;6.87 0.35;6.85 0.33;4.06 0.56;4.04 1.74;4.02 1.77;4.00 0.62;3.92 0.37;3.86 1.00;3.35 143.04;3.34 312.18;2.79 0.68;2.78 0.62;2.74 0.37;2.73 0.35;2.68 0.56;2.67 0.68;2.67 0.49;2.54 0.38;2.53 0.96;2.51 54.21;2.50 72.95;2.34 0.46;2.33 0.64;2.33 0.79;2.33 0.64;2.32 0.46;2.07 1.95;2.04 0.35;2.01 16.00;1.99 8.08;1.36 0.69;1.24 0.46;1.19 2.26;1.17 4.49;1.16 2.17;0.88 0.50;0.86 0.36;0.00 9.11 |
| Ia-12 | [DMSO-D₆] 7.01 4.28;4.03 0.82;4.02 0.83;3.79 0.35;3.37 0.42;3.34 331.71;3.33 0.37;3.32 2.34;2.79 0.50;2.62 0.35;2.61 0.48;2.61 0.35;2.52 0.66;2.52 0.84;2.52 0.90;2.51 23.83;2.51 51.40;2.50 70.93;2.50 51.33;2.50 23.51;2.44 0.53;2.39 0.35;2.39 0.53;2.38 0.38;2.36 0.58;2.33 0.49;2.31 0.74;2.28 16.00;2.19 0.55;1.99 3.80;1.97 7.19;1.19 0.93;1.17 1.84;1.16 0.90;0.01 0.74;0.00 23.54;-0.01 0.71 |
| Ia-13 | [CDCl₃] 7.26 51.97;7.09 0.34;7.04 0.43;6.93 0.64;6.92 0.56;3.73 0.51;3.72 0.51;3.49 0.37;3.30 1.06;3.29 1.10;3.29 0.94;3.28 0.80;3.01 1.54;2.42 16.00;2.36 0.92;1.57 10.32;1.26 0.76;1.25 1.28;1.23 0.63;0.01 0.86;0.00 28.20;-0.010.93 |
| Ia-14 | 1H-NMR(D6-DMSO): 7,67(s,1H), 7,15(s,1H), 4,16(q,2H) |
| Ia-15 | 1H-NMR(D6-DMSO): 8,30(breit,1H), 7,61(s,1H), 7,20(s,1H), 4,14(q,2H), 2,62(breit,3H) |
| Ia-17 | 1H-NMR(D6-DMSO): 7,15(d,1H), 7,10(breit, 2H), 6,92(d,1H), 3,90(q,2H), 2,01(s,3H) |
| Ia-18 | [CDCl₃] 7.26 18.05;7.26 0.39;7.25 0.35;7.24 0.34;7.04 0.64;6.94 0.46;6.93 1.00;6.92 0.76;6.91 0.76;6.84 0.35;6.82 0.33;5.26 0.47;3.45 1.08;3.41 0.41;3.39 0.32;3.30 1.56;3.28 1.39;2.47 0.74;2.42 16.00;1.58 15.92;1.29 1.95;1.26 0.51;1.25 0.43;1.25 0.48;1.09 0.37;0.00 9.63;-0.010.33 |
| Ia-19 | 1H-NMR (CDCl3): 7,58 (d,1H), 7,13 (s,1H), 6,85 (d,1H), 5,31 (breit,1H), 3,45 (q,2H), 2,93 (breit,3H) |
| Ia-21 | 1H-NMR(D6-DMSO): 7,17(d,1H), 7,09(d,1H), 3,91(q,2H), 2,34(s,3H) |
| Ia-23 | 1H-NMR(D6-DMSO): 7,02(s,1H), 6,79(s,1H), 3,83(q,2H), 2,87(breit,6H), 2,28(s,3H), 1,96(s,3H) |
| Ia-24 | 1H-NMR(D6-DMSO): 7,09(d,1H), 6,86(breit,1H), 3,89(q,2H), 2,90(breit,3H), 2,00(s,3H) |
| Ia-25 | 1H-NMR (CDCl3): 7,72 (d,1H), 7,24 (m,1H), 6,96-6,99 (m,1H), 4,98 (breit,2H), 3,48(q,2H) |
| Ia-26 | [CDCl₃] 7.26 29.38;6.94 1.54;6.92 1.55;3.73 0.38;3.72 0.38;3.49 0.34;3.33 0.70;3.32 0.83;2.47 0.43;2.42 16.00;1.57 6.79;1.26 0.58;1.25 1.07;1.23 0.54;0.48 0.53;0.43 0.39;0.010.45;0.00 15.80;-0.01 0.53 |

| **Beispielsnummer** | **1H-NMR Daten** |
|---|---|
| Ib-01 | 1H-NMR(D6-DMSO): 7,29(d,1H), 7,23(d,1H), 4,02-4,14(m,2H), 3,01(breit,6H), 2,31(s,3H) |
| Ib-02 | 1H-NMR(D6-DMSO): 8,30(breit,1H), 7,29(d,1H), 7,22(d,1H), 4,08-4,14(m,2H), 3,99(breit,2H), 2,31(s,3H), 1,11(breit,3H) |
| Ib-03 | 1H-NMR(D6-DMSO): 8,02(breit,1H), 7,28(d,1H), 7,21(d,1H), 4,08-4,14(m,2H), 3,96-4,00(m,1H), 2,31(s,3H), 1,12-1,17(breit,6H) |
| Ib-04 | 1H-NMR(D6-DMSO): 8,01(breit,1H), 7,00-7,20(m,2H), 3,70-4,10(m,2H), 2,80(breit,3H), 2,27(s,3H), 2,07(s,3H) |
| Ib-05 | [DMSO-D₆] 7.76 0.68;7.75 0.68;7.48 0.42;7.46 0.41;7.27 5.26;7.26 5.26;7.09 0.34;7.02 0.49;4.32 0.34;4.30 0.38;4.27 0.47;4.26 0.80;4.24 1.46;4.22 2.04;4.20 |
| | 1.69;4.18 0.75;3.35 821.56;3.34 2.15;3.33 2.32;3.17 0.58;3.16 0.56;2.81 0.80;2.73 0.36;2.62 0.57;2.62 1.01;2.62 1.31;2.61 0.98;2.61 0.52;2.54 1.16;2.52 3.60;2.52 4.50;2.52 5.20;2.51 63.77;2.51 130.55;2.50 175.10;2.50 122.46;2.50 53.43;2.48 0.53;2.39 0.67;2.39 1.11;2.39 1.41;2.38 1.11;2.38 0.73;2.37 6.18;2.33 0.38;2.31 0.37;2.27 4.25;2.11 16.00;2.09 5.30;2.08 1.22;2.02 0.44;1.91 0.99;1.23 0.51;1.14 1.46;0.01 0.51;0.00 12.92 |
| Ib-06 | 1H-NMR(D6-DMSO): 7,28(d,1H), 7,22(d,1H), 4,10-4,14(m,1H), 3,90-4,09(m,1H), 3,10-3,30(breit,2H), 2,31(s,3H), 1,51-1,56(breit,2H), 0,84-0,91(breit,3H) |
| Ib-07 | 1H-NMR(D6-DMSO): 7,30(d,1H), 7,22(d,1H), 3,99-4,15(m,2H), 2,66-2,73(breit,3H),2,31(s,3H) |
| Ib-08 | [DMSO-D₆] 8.43 0.75;7.39 0.32;7.22 1.80;7.20 1.79;4.37 0.45;4.13 0.35;4.11 0.82;4.09 0.84;4.07 0.47;4.05 0.32;4.03 0.53;4.02 0.49;3.45 0.50;3.44 0.51;3.44 0.56;3.43 0.58;3.43 0.36;3.42 0.38;3.40 0.48;3.40 0.57;3.39 1.02;3.38 1.14;3.35 1478.79;3.33 18.72;2.62 0.34;2.62 0.79;2.62 1.07;2.61 0.78;2.61 0.36;2.54 0.67;2.52 1.59;2.52 2.06;2.52 2.16;2.51 57.91;2.51 127.88;2.50 173.56;2.50 123.42;2.50 53.41;2.39 0.33;2.39 0.75;2.39 1.04;2.38 0.74;2.38 0.33;2.31 16.00;2.08 2.40;1.99 1.93;1.23 0.45;1.19 0.50;1.17 1.09;1.16 0.49;1.07 0.79;1.05 1.55;1.04 0.73;0.42 0.41;0.41 0.44;0.01 0.39;0.00 13.49;-0.01 0.41 |
| Ib-09 | 1H-NMR(D6-DMSO): 7,12(s,1H), 7,03(s,1H), 4,03-4,07(m,2H), 2,91(breit,6H), 2,27(s,3H), 2,07(s,3H) |
| Ib-10 | 1H-NMR (CDCl3): 7,63-7,71 (m,2H), 7,07 (d,1H), 5,63 (breit,1H), 3,25-3,58 (m,2H), 2,94 (d,3H) |
| Ib-11 | 1H-NMR(D6-DMSO): 7,37(d,1H), 7,28(d,1H), 4,03-4,10(m,2H), 2,34(s,3H) |
| Ib-12 | 1H-NMR(D6-DMSO): 7,19(s,1H), 7,17(s,1H), 3,96-4,04(m,2H), 2,30(s,3H), 2,07(s,3H) |
| Ib-13 | 1H-NMR(D6-DMSO): 7,31(d,1H), 7,10(d,1H), 4,23-4,29(m,1H), 4,13-4,18(m,1H), 2,10(s,3H) |
| Ib-14 | 1H-NMR(D6-DMSO): 7,87(s,1H), 7,54(breit,2H), 7,33(s,1H), 4,10-4,18(m,2H) |
| Ib-15 | 1H-NMR(D6-DMSO): 7,27(d,1H), 6,98(d,1H), 4,23(m,2H), 2,94(breit,6H), 2,09(s,3H) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Wenn auf Grund der Rundung des δ-Wertes auf zwei Nachkommastellen fallweise Signale mit gleichem δ-Wert auftreten, ergeben deren Intensitäten nach Addition dasselbe Bild, das auch in einem Ausdruck eines klassischen NMR's im Bereich dieses δ-Wertes beobachtet werden kann.

### Anwendungsbeispiele

### Phaedon -Test (PHAECO Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 500 g/ha: Ia-02, Ia-04, Ia-05, Ia-13, Ia-14, Ia-15, Ia-18, Ia-19, Ib-02, Ib-10, Ib-11, Ib-14, Ib-16

**Tetranychus - test, OP-resistent (TETRUR Spritzbehandlung)**

| | |
|---|---|
| Lösungsmittel: | 78,0 Gewichtsteile Aceton |
| | 1,5 Gewichtsteile Dimethylformamid |
| Emulgator : | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 80 % bei einer Aufwandmenge von 500g/ha: Ia-09, Ia-17, Ib-04, Ib-05

Bei diesem Test zeigen z. B. die folgenden Verbindungen der Herstellungsbeispiele eine Wirkung von 100 % bei einer Aufwandmenge von 500g/ha: Ia-02, Ia-03, Ia-05, Ia-07, Ia-12, Ia-13, Ia-15, Ia-18, Ia-23, Ia-26, Ib-02, Ib-03, Ib-06, Ib-07, Ib-08, Ib-09, Ib-12, Ib-13, Ib-15

### Meloidogyne incognita- Test (MELGIN)

Lösungsmittel: 80,0 Gewichtsteile Aceton

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, Meloidogyne incognita Ei- Larven- Suspension und Salatsamen gefüllt. Die Salatsamen keimen und die Pflänzchen entwickeln sich. An den Wurzeln entwickeln sich die Gallen.

Nach 14 Tagen wird die nematizide Wirkung an hand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigt z. B. die folgende Verbindung der Herstellungsbeispiele eine Wirkung von 90 % bei einer Aufwandmenge von 20 ppm : Ia-07

Bei diesem Test zeigt z. B. die folgende Verbindung der Herstellungsbeispiele eine Wirkung von 100 % bei einer Aufwandmenge von 20 ppm : Ib-02

### Lucilia cuprina (48h)

Lösungsmittel: Dimethylsulfoxid

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration. Etwa 20 Lucilia cuprina-Larven werden in ein Teströhrchen gebracht, welches ca. 1 cm³ Pferdefleisch und 0.5 ml der zu testende Wirkstoffzubereitung enthält. Nach 48 Stunden wird die Wirksamkeit der Wirkstoffzubereitung als % Larvenmortalität ermittelt.

Bei diesem Test zeigen z.B. die folgenden Verbindungen der Herstellungsbeispiele eine Wirkung von 100% bei einer Aufwandmenge von 100 ppm: Ia-21, Ib-11

### Boophilus microplus -Test (BOOPMI Injektion)

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigt z.B. die folgende Verbindung der Herstellungsbeispiele eine Wirkung von 80 % bei einer Aufwandmenge von 20µg / Tier : Ia-04

Bei diesem Test zeigen z.B. die folgenden Verbindungen der Herstellungsbeispiele eine Wirkung von 100 % bei einer Aufwandmenge von 20µg / Tier : Ia-01, Ia-21, Ia-22, Ib-01, Ib-10, Ib-11, Ib-14, Ib-16

Wenn nicht anders erwähnt, wurde die Testlösung in den folgenden Beispielen wie folgt vorbereitet:

Man mischt 1 Gewichtsteil Wirkstoff mit den angegebenen Mengen eines Lösungsmittel und Emulgator - Gemisches (3 Gewichtsteile Dimethylformamide als Lösungsmittel und 1Gewichtsteil Polyoxyethylene alkyl phenyl ether als Emulgator) und verdünnt die Lösung mit Wasser auf die gewünschte Konzentation

### Test gegen Spinnmilbe (Tetranychus urticae)

50 bis 100 erwachsene Spinnmilben werden auf die Blätter einer Kidney-Bohnenpflanze im Zweiblattstadium gesetzt, die in einem Topfen mit 6cm Durchmesser wächst.

Nach einem Tag wird die Pflanze mit Hilfe einer Farbspritzpistole mit der Wirkstoffzubereitung der gewünschten Konzentration in ausreichender Menge gespritzt und in einem Gewächshaus aufgestellt. Nach 7 Tagen wird die akarizide Wirkung ermittelt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100 % bei einer Wirkstoffkonzentation von 100ppm:
Ia-21, Ia-01, Ib-11, Ib-01, Ia-06, Ia-14, Ia-15, Ib-14, Ia-20, Ia-25, Ia-19, Ib-16 und Ib-10

### Test gegen den Kürbis-Blattkäfer (Aulacophora femoralis)

Gurkenblätter werden in die Testlösung mit der entsprechenden Konzentration getaucht und dann an der Luft getrocknet. Danach werden die Blätter in eine Plastikschale mit steriler Erde und fünf, im zweiten Larvenstadium befindlichen, Aulacophora femoralis Larven gesteckt Die Schalen werden in einem klimatisierten Raum bei 25°C aufgestellt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von 100 % bei einer Wirkstoffkonzentation von 500ppm:
Ia-21, Ib-11, Ib-01, Ib-14, Ib-16 und Ib-10

## Patentansprüche

1. N-Arylamidine-substituierte Trifluoroethylsulfid-Derivate der Formel (I) 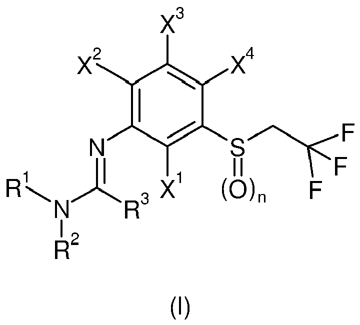 in welcher
n für die Zahl 0, 1 oder 2 steht,
X¹, X², X³, X⁴ unabhängig von einander für Wasserstoff, Halogen, Hydroxy, Amino, OCN, SCN, SF₅, Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Alkoxycarbonylalkyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl, Dialkylaminothiocarbonyl,
oder für gegebenenfalls substituiertes Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy Hetarylthio, Hetarylsulfinyl, Hetarylsulfonyl,
oder für gegebenenfalls substituiertes gesättigtes oder ungesättigtes Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl,
oder für NR⁴R⁵, wobei R⁴ und R⁵ unabhängig voneinander für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl, Alkoxycarbonyl stehen oder R⁴ und R⁵ gemeinsam mit dem N-Atom, an dem sie gebunden sind, einen gegebenenfalls substituierten und gegebenenfalls durch Heteroatome aus der Reihe O, S oder N unterbrochenen gesättigten oder ungesättigten fünf- bis achtgliedrigen Ring bilden können, stehen,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcarbonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes, gegebenenfalls durch Heteroatome aus der Reihe O, S oder N unterbrochenes Cycloalkyl, Cycloalkoxy, Cycloalkylalkyl oder Cycloalkylalkoxy steht,
oder, X² und X³, oder X³ und X⁴, bilden einen 5 oder 6-gliedrigen Ring mit den C-Atome an die sie gebunden sind aus, der gegebenenfalls subsitutiert ist und gegebenenfalls durch Heteroatome aus der Reihe O, S, N oder CO unterbrochen ist.
R³ für Wasserstoff, (C₂-C₈)Alkyl, Cyano, Halogenalkyl, Alkoxyalkyl, Cyanoalkyl, Alkylthioalkyl, Alkenyl, Halogenalkenyl, Alkinyl, Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy steht,
R¹ und R² unabhängig voneinander für Wasserstoff, Cyano, Alkyl, Halogenalkyl, Alkoxy, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, für gegebenenfalls substituierte Alkylcarbonyl, Alkoxycarbonyl, Arylcarbonyl, Hetarylcarbonyl, Aryloxycarbonyl, Hetaryloxycarbonyl, Alkylsulfinyl, Halogenalkylsulfinyl, Arylsulfinyl, Arylalkylsulfinyl, Hetarylsulfinyl, Hetarylalkylsulfinyl, Alkylsulfonyl, Halogenalkylsulfonyl, Arylsulfonyl, Arylalkylsulfonyl, Hetarylsulfonyl, Hetarylalkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen, wobei m für die Zahl 1, 2, 3 oder 4 steht, oder
R¹ und R² auch zusammen mit dem N-Atom, an das sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann, oder
R¹ und R³ auch zusammen mit den Atomen, an die sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann.

2. Verbindungen der allgemeinen Formel (I) gemäß Anspruch 1, wobei
n für die Zahl 0, 1 oder 2 steht,
X¹, X², X³, X⁴ unabhängig von einander für Wasserstoff, Halogen, Hydroxy, Amino, OCN, SCN, SF₅, Tri-(C₁-C₆)alkylsilyl,(C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₄)-alkoxy, (C₁-C₆)Alkoxycarbonyl-(C₁-C₄)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₇)Alkoxycarbonyl-(C₁-C₆)alkoxy, (C₁-C₆)Alkoxy-(C₁-C₆)alkoxy, (C₁-C₇)Alkylhydroxyimino, (C₁-C₇)Alkoxyimino, (C₁-C₆)Alkyl-(C₁-C₇)alkoxyimino, (C₁-C₆)Halogenalkyl-(C₁-C₇)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₇)Alkylcarbonyl, (C₁-C₇)Halogenalkylcarbonyl, Carboxyl, (C₁-C₇)Alkylcarbonyloxy, (C₁-C₇)Alkoxycarbonyl, (C₁-C₇)Halogenalkoxycarbonyl, (C₁-C₇)Alkoxycarbonyl-(C₁-C₆)alkyl, Aminocarbonyl, (C₁-C₇)Alkylaminocarbonyl, Di-(C₁-C₇)alkyl-aminocarbonyl, (C₁-C₇)Alkenylaminocarbonyl, Di-(C₁-C₇)-alkenylaminocarbonyl, (C₃-C₈)Cycloalkylaminocarbonyl, (C₁-C₆)Alkylsulfonylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl,
oder für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl substituiertes gegebenenfalls durch ein oder zwei Heteroatome aus der Reihe O, S oder N unterbrochenes, gegebenenfalls gesättigtes oder ungesättigtes (C₃-C₈)Cycloalkyl, (C₃-C₈)Cycloalkoxy, (C₃-C₈)Cycloalkyl(C₁-C₆)alkyl oder (C₃-C₈)Cycloalkyl-(C₁-C₆)alkoxy substituiertes Phenyl-(C₁-C₆)alkyl, Phenoxy, Phenyl-(C₁-C₄)alkyloxy, Phenoxy-(C₁-C₄)alkyl, Phenylthio, Phenylthio-(C₁-C₄)alkyl, Phenylsulfinyl, Phenylsulfonyl, Hetaryl-(C₁-C₆)alkyl, Hetaryloxy, Hetaryl-(C₁-C₄)alkyloxy, Hetarylthio, Hetarylsulfinyl, Hetarylsulfonyl, gegebenenfalls gesättigtes oder ungesättigtes (C₃-C₈)Cycloalkyl-(C₁-C₆)alkyl, (C₃-C₈)Cycloalkyloxy, (C₃-C₈)Cycloalkyl-(C₁-C₆)alkoxy, (C₃-C₈)Cycloalkylthio, (C₃-C₈)Cycloalkyl-(C₁-C₆)alkylthio, (C₃-C₈)Cycloalkylsulfinyl, (C₃-C₈)Cycloalkyl-(C₁-C₆)alkylsulfinyl, (C₃-C₈)Cycloalkylsulfonyl, (C₃-C₈)Cycloalkyl-(C₁-C₆)alkylsulfonyl,
oder für NR⁴R⁵, wobei R⁴ und R⁵ unabhängig voneinander für Wasserstoff, Cyano, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkyl-(C₁-C₆)thioalkyl, (C₂-C₈)Alkenyl, (C₂-C₈)Halogenalkenyl, (C₂-C₈)Cyanoalkenyl, (C₂-C₈)Alkinyl, (C₂-C₈)Halogenalkinyl, (C₂-C₈)Cyanoalkinyl, Acyl, (C₁-C₇)Alkoxycarbonyl stehen oder R⁴ und R⁵ gemeinsam mit dem N-Atom, an dem sie gebunden sind, einen gegebenenfalls gleich oder verschieden durch Halogen, Cyano, Hydroxy, Amino, (C₁-C₆)Alkyl, (C₁-C₆)Halogenalkyl, (C₁-C₆)Alkoxy, (C₁-C₆)Halogenalkoxy, oder durch gegebenenfalls gleich oder verschieden durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl oder (C₃-C₈)Cycloalkyl(C₁-C₆)alkyl, substituierten und gegebenenfalls durch Heteroatome aus der Reihe O, S oder N unterbrochenen gesättigten oder ungesättigten fünf- bis siebengliedrigen Ring bilden können, stehen,
oder für einen (C₃-C₈)Cycloalkyl, (C₃-C₈)Cycloalkoxy, (C₃-C₈)Cycloalkylthio, (C₃-C₈)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden substituiert ist mit Halogen, Cyano, Nitro, (C₁-C₆)Alkyl, (C₁-C₆)Halogenalkyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, oder durch gegebenenfalls durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl(-C₁-C₆)alkyl oder (C₃-C₈)Cycloalkyl-(C₁-C₆)alkoxy steht,
oder, X² und X³, oder X³ und X⁴, können die folgenden 5 oder 6-gliedrigen Ring ausbilden, die gegebenenfalls gleich oder verschieden durch Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, subsitutiert sind, 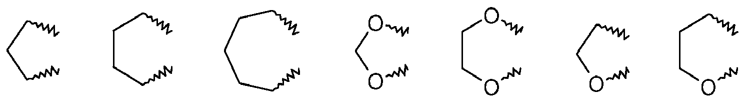 oder X² und X³, oder X³ und X⁴, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, (C₁-C₆)Alkoxy(C₁-C₆)alkyl, (C₁-C₆)Alkylthio, (C₁-C₆)Alkylsulfonyl, Amino, (C₁-C₆)Alkylamino, Di-(C₁-C₆)-alkylamino oder (C₃-C₈)Cycloalkylamino, 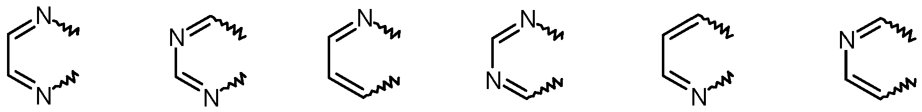 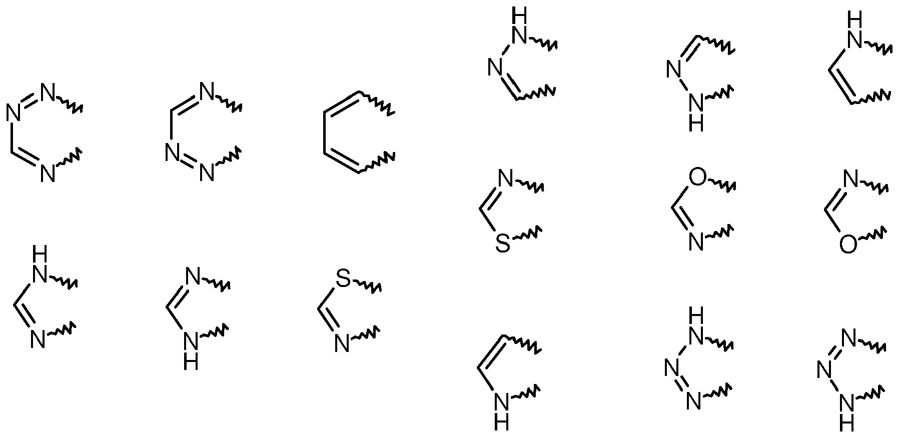 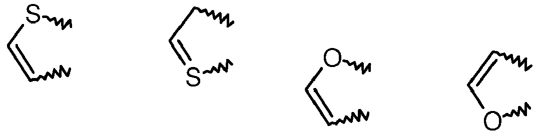 R³ für Wasserstoff, (C₂-C₆)Alkyl, Cyano, (C₁-C₆)Halogenalkyl, (C₁-C₆)Alkoxy-(C₁-C₆)alkyl, (C₁-C₆)Cyanoalkyl, (C₁-C₆)Alkylthio-(C₁-C₆)alkyl, (C₂-C₆)Alkenyl, (C₂-C₆)Halogenalkenyl, (C₂-C₆)Alkinyl, (C₂-C₆)Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₆)alkylaminocarbonyl, Aminothiocarbonyl, (C₁-C₆)Alkylaminothiocarbonyl, Di-(C₁-C₆)alkylaminothiocarbonyl, (C₃-C₆)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl, oder substituiert mit gegebenenfalls gleich oder verschieden durch Halogen, Cyano, Nitro, Hydroxy, Amino, Carboxy, (C₁-C₆)Alkyl, (C₁-C₆)Halogenalkyl, (C₁-C₆)Alkoxy, (C₁-C₆)Halogenalkoxy substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
R¹ und R² unabhängig voneinander für Wasserstoff, Cyano, (C₁-C₆)Alkyl, (C₁-C₆)Halogenalkyl, (C₁-C₆)Alkoxy, (C₁-C₆)Cyanoalkyl, (C₁-C₆)Hydroxyalkyl, (C₁-C₆)Alkoxy-(C₁-C₆)alkyl, (C₁-C₆)Alkylthio-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl,
für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylamino, Di-(C₁-C₆)alkyl-amino substituierte (C₁-C₆)Alkylcarbonyl, (C₁-C₇)Alkoxycarbonyl, Arylcarbonyl, Hetarylcarbonyl, Aryloxycarbonyl, Hetaryloxycarbonyl, (C₁-C₆)Alkylsulfinyl, (C₁-C₆)Halogenalkylsulfinyl, Arylsulfinyl, Aryl(C₁-C₆)alkylsulfinyl, Hetarylsulfinyl, Hetaryl(C₁-C₆)alkylsulfinyl, (C₁-C₆)Alkylsulfonyl, (C₁-C₆)Halogenalkylsulfonyl, Arylsulfonyl, Aryl(C₁-C₆)alkylsulfonyl, Hetarylsulfonyl, Hetaryl(C₁-C₆)alkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)-alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)-alkylaminosulfonyl, oder mit gegebenenfalls substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkylamino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder gleich oder verschieden mit gegebenenfalls durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen, wobei m für die Zahl 1, 2 oder 3 steht, oder
R¹ und R² zusammen mit den Atomen, an das sie gebunden sind, einen gesättigten oder ungesättigten und gegebenenfalls einfach oder mehrfach durch (C₁-C₄)Alkyl, (C₁-C₄)Alkoxy, Halogen, (C₁-C₄)Halogenalkyl substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe stehen,
R¹ und R³ zusammen mit den Atomen, an die sie gebunden sind, einen gesättigten oder ungesättigten und gegebenenfalls einfach oder mehrfach gleich oder verschieden durch (C₁-C₄)Alkyl, (C₁-C₄)Alkoxy, Halogen, (C₁-C₄)Halogenalkyl, (C₂-C₄)Alkandiyl, (C₂-C₄)Alkendiyl, Butandienyl (wobei Alkandiyl, Alkendiyl und Butandienyl gegebenenfalls durch (C₁-C₄)Alkyl, (C₁-C₄)Alkoxy, Halogen, (C₁-C₄)Halogenalkyl substituiert sein können und/oder gegebenenfalls durch mindestens ein Sauerstoff- oder/und Stickstoffatom unterbrochen sein können) substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe enthalten kann,

3. Verbindungen der allgemeinen Formel (I) gemäß einem der Ansprüche 1 oder 2, wobei
n für die Zahl 0 oder 1 steht,
X¹, X², X³, X⁴ unabhängig von einander für Wasserstoff, Fluor, Chlor, Brom, Jod, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, Aminothiocarbonyl,
oder für gegebenenfalls einfach oder zweifach gleich oder verschieden durch Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, oder durch gegebenenfalls durch ein Heteroatom aus der Reihe O, S oder N unterbrochenes, gegebenenfalls gesättigtes oder ungesättigtes (C₃-C₆)Cycloalkyl substituiertes Phenyl-(C₁-C₄)alkyl, Phenoxy, Hetaryl-(C₁-C₄)alkyl, Hetaryloxy, gegebenenfalls gesättigtes oder ungesättigtes (C₃-C₆)Cycloalkyl-(C₁-C₄)alkyl, (C₃-C₆)Cycloalkyloxy, (C₃-C₆)Cycloalkyl-(C₁-C₄)alkoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl, der gegebenenfalls einfach oder zweifach, gleich oder verschieden substituiert ist mit Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, (C₃-C₆)Cycloalkyl steht,
oder, X² und X³, oder X³ und X⁴, können die folgenden 5 oder 6-gliedrigen Ring ausbilden, die gegebenenfalls einfach bis vierfach durch Fluor oder (C₁-C₄)Alkyl subsitutiert sind, 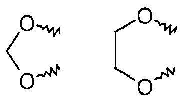 oder X² und X³, oder X³ und X⁴, können die folgenden anellierten Ringe ausbilden, die gegebenenfalls einfach oder zwei, gleich oder verschieden substituiert sind, wobei die Substituenten unabhängig voneinander ausgewählt sein können aus Wasserstoff, Fluor, Chlor, Brom, Cyano, (C₁-C₄)Alkyl, (C₃-C₆)Cycloalkyl, (C₁-C₄)Halogenalkyl, (C₁-C₄)Alkoxy, (C₁-C₄)Halogenalkoxy, 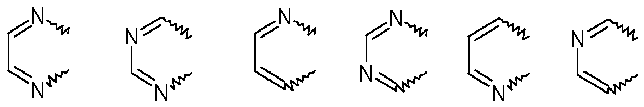 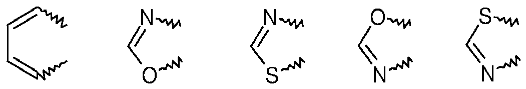 R³ für Wasserstoff, (C₂-C₄)Alkyl, Cyano, (C₁-C₄)Halogenalkyl, (C₁-C₄)Alkoxy-(C₁-C₄)alkyl, (C₁-C₄)Cyanoalkyl,
oder für einen 3- bis 6-gliedrigen gesättigten, teilgesättigten oder aromatischen Ring, der gegebenenfalls ein bis zwei Heteroatome aus der Reihe O, S oder N enthalten kann und der gegebenenfalls einfach oder dreifach, gleich oder verschieden substituiert ist mit Fluor, Chlor, Brom, Jod, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, oder substituiert mit (C₃-C₆)Cycloalkyl steht,
R¹ und R² unabhängig voneinander für Wasserstoff, Cyano, (C₁-C₄)Alkyl, (C₁-C₄)Halogenalkyl, (C₁-C₄)Alkoxy, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Alkinyl,
für gegebenenfalls einfach bis dreifach unabhängig voneinander durch Fluor, Chlor, Brom, Cyano, Nitro, (C₁-C₄)Alkyl, (C₁-C₄)Halogenalkyl, (C₂-C₄)Alkenyl, (C₂-C₄)Halogenalkenyl, (C₂-C₄)Alkinyl, (C₁-C₄)Alkoxy, (C₁-C₄)Halogenalkoxy, (C₁-C₄)Alkylthio, (C₁-C₄)Alkylsulfinyl, (C₁-C₄)Alkylsulfonyl, (C₁-C₄)Alkylamino, Di-(C₁-C₄)alkyl-amino substituiertes (C₁-C₄)Alkylcarbonyl, (C₁-C₅)Alkoxycarbonyl, Arylcarbonyl, Thiophenylcarbonyl, Pyridylcarbonyl, Pyrimidylcarbonyl, Thiazolylcarbonyl, Pyrazolylcarbonyl, (C₁-C₄)Alkylsulfinyl, (C₁-C₄)Halogenalkylsulfinyl, Arylsulfinyl, Aryl(C₁-C₄)alkylsulfinyl, Hetarylsulfinyl, Hetaryl(C₁-C₄)alkylsulfinyl, (C₁-C₄)Alkylsulfonyl, (C₁-C₄)Halogenalkylsulfonyl, Arylsulfonyl, Aryl(C₁-C₄)alkylsulfonyl, Hetarylsulfonyl, Hetaryl(C₁-C₄)alkylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen,
oder für -(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen, wobei m für die Zahl 1 oder 2 steht, oder
R¹ und R² zusammen mit dem N-Atom, an das sie gebunden sind, einen gesättigten oder ungesättigten und gegebenenfalls einfach oder vierfach durch Fluor, Chlor, (C₁-C₄)Alkyl, (C₁-C₄)Alkoxy, (C₁-C₄)Halogenalkyl substituierten 3- bis 6-gliedrigen Ring bilden können, der gegebenenfalls durch ein weiteres Heteroatom aus der Reihe Schwefel, Sauerstoff und Stickstoff enthalten kann und/oder mindestens eine Carbonylgruppe stehen,
R¹ und R³ zusammen mit den Atomen, an die sie gebunden sind, einen gesättigten oder ungesättigten und gegebenenfalls einfach oder mehrfach durch Methyl, Ethyl, Methoxy, Ethoxy, Fluor, Chlor, Trifluoromethyl, (C₂-C₄)Alkandiyl, (C₂-C₄)Alkendiyl, Butandienyl (wobei Butandienyl gegebenenfalls einfach oder zweifach durch Methyl, Fluor, Chlor, Brom, Methoxy oder Trifluoromethyl substituiert sein können und/oder gegebenenfalls durch mindestens ein Sauerstoff- oder/und Stickstoffatom unterbrochen sein können) substituierten 5- bis 6-gliedrigen Ring bilden können, der gegebenenfalls ein weiteres Schwefel- oder Sauerstoff- oder Stickstoffatom und/oder eine Carbonylgruppe enthalten kann.

4. Verbindungen der allgemeinen Formel (I) gemäß einem der Ansprüche 1 bis 3, wobei n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Difluoromethoxy, Trifluoromethoxy, OCH₂CF₃,
insbesonders stehen X² und X⁴ für folgende Kombinationen X²/X⁴: F/F, Cl/Cl, F/Cl, Cl/F, BrBr, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl,
oder für Aryl steht, insbesondere für Phenyl steht,
R¹ für Wasserstoff, Methyl, Ethyl, Propyl, Butyl, sec-Butyl, iso-Propyl, tert-Butyl, (2,2,2)Trifluoroethyl, (2,2)Difluoromethyl, Methoxy, Ethoxy, Methoxymethyl, Methoxyethyl,
oder für Aryl steht, welches gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere wird Phenyl explizit genannt: welches gegebenenfalls mit den unter ganz besonders bevorzugt genannten Subtituenten substituiert sein kann,
oder für -(CH₂)ₘ-R⁶, wobei R⁶ für Aryl steht, welches gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere wird Phenyl für R⁶ explizit genannt, welches gegebenenfalls mit den unter ganz besonders bevorzugt genannten Subtituenten substituiert sein kann,
R² für Wasserstoff, Methyl oder Ethyl steht, oder
R¹ und R² zusammen mit dem N-Atom, an das sie gebunden sind, einen gesättigten oder ungesättigten 5- bis 6-gliedrigen Ring bilden können, der gegebenenfalls ein Sauerstoffatom enthalten kann, insbesondere werden folgende Ringe explizit genannt, 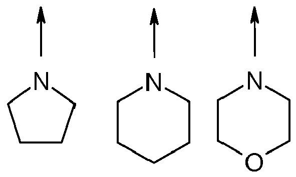 in denen das N-Atom, an das R¹ und R² gebunden sind, den Cyclus darstellen und der Pfeil zum Rest des Moleküls zeigt,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls eine Carbonylgruppe enthalten kann, insbesondere stehen R¹ und R³ zusammen mit den Atomen, an die sie gebunden sind für folgende Gruppe, 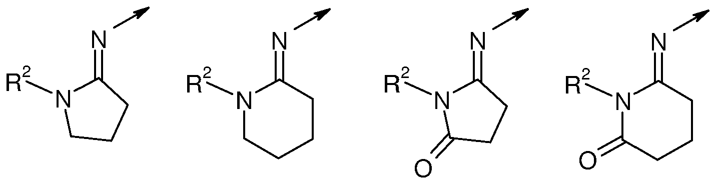 wobei der Pfeil zum Rest des Moleküls zeigt.

5. Verbindungen der allgemeinen Formel (1) gemäß einem der Ansprüche 1 bis 3, wobei n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Vinyl, Ethinyl,Methoxy, Ethoxy, Difluoromethoxy, Trifluoromethoxy, OCH₂CF₃,Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders stehen X² und X⁴ für folgende Kombinationen X²/X⁴: F/F, Cl/Cl, F/Cl, Cl/F, BrBr, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ für Cyano, Cyanomethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für Morpholin, Oxetan, Thienyl oder Pyridyl steht, die gegebenenfalls einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für Cyclopropyl, Cyclobutyl, Cyclopentyl, Cyclohexyl, steht, die einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für Aryl steht, insbesondere für Phenyl steht, welches einfach oder mehrfach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für -(CH₂)-R⁶, wobei R⁶ für Cyclopropyl oder Pyridyl steht, die gegebenenfalls einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder wobei R⁶ für Aryl steht, insbesondere für Phenyl steht, welches einfach oder mehrfach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der ein oder zwei weitere Heteroatome aus der Reihe Schwefel oder Stickstoff enthält,
oder zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 4-gliedrigen Ring bilden können, der gegebenenfalls ein weiteres Heteroatom aus der Reihe Sauerstoff, Schwefel oder Stickstoff enthält
insbesondere werden folgende Ringe explizit genannt, 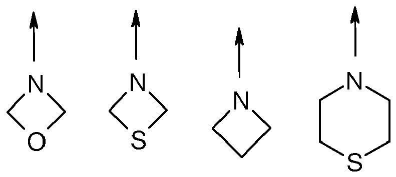 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

6. Verbindungen der allgemeinen Formel (I) gemäß einem der Ansprüche 1 bis 3,
wobei
n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Vinyl, Ethinyl,Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ für Methoxymethyl, Methoxyethyl, Cyanomethyl, Allyl, Butenyl,
oder für Cyclopropyl, Pyridyl, Pyrimidyl, Thiazolyl, Pyrazolyl oder Thienyl steht, die gegebenenfalls einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert sein können,
oder für Aryl, insbesondere für Phenyl steht, welches einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert ist,
R¹ und R² unabhängig voneinander für Wasserstoff, Cyano, Methyl, Ethyl, Propyl, Butyl, *sec-*Butyl, iso-Propyl, tert-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Morpholin, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 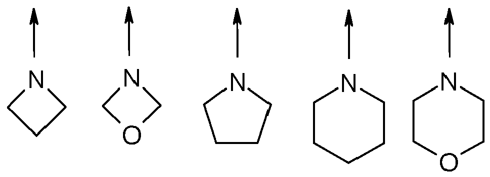 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

7. Verbindungen der allgemeinen Formel (I) gemäß einem der Ansprüche 1 bis 3, wobei n für die Zahl 0 oder 1 steht,
X¹ und X³ für Wasserstoff stehen,
X² und X⁴ unabhängig voneinander für Vinyl, Ethinyl, Methoxy, Ethoxy, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ und R² unabhängig voneinander für Wasserstoff, Cyano, Methyl, Ethyl, Propyl, Butyl, sec-Butyl, iso-Propyl, tert-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen, oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Morpholin, Oxetan, Phenyl, Thienyl und Pyridyl, die gegebenenfalls mit den unter ganz besonderen bevorzugten genannten Subtituenten substituiert sein können,
oder für -(CH2)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 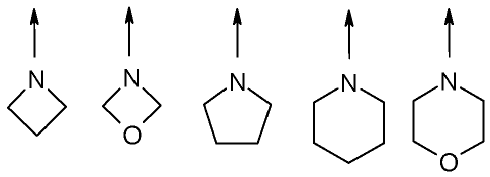 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 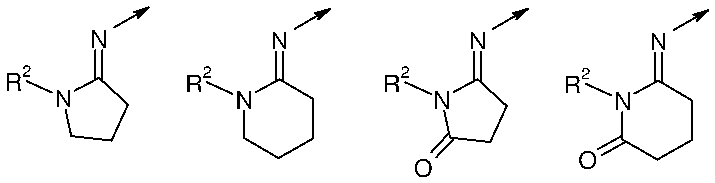 wobei der Pfeil zum Rest des Moleküls zeigt.

8. Verfahren zur Herstellung von Verbindungen der Formel (I) gemäß einem der Ansprüche 1 bis 7, **dadurch gekennzeichnet, dass** man
(A) Aniline der Formel (VII) 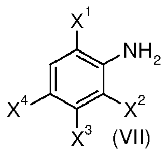 in Gegenwart von Säuren, Säureanhydriden oder Säurechloriden der Formel (VIII) 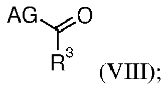 wobei AG für eine Abgangsgruppe steht, umsetzt zu Aniliden der allgemeinen Formel (VI) 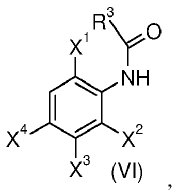
(B) Anilide der Formel (VI) durch Chlorsulfonierung mit Chlorsulfonsäure in Sulfonylchloride der allgemeinen Formel (V) umsetzt, 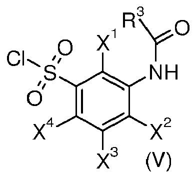
(C) Sulfonylchloride der Formel (V) durch Reduktion mit Eisen in Salzsäure oder Iodid in Disulfide der allgemeinen Formel (IV) 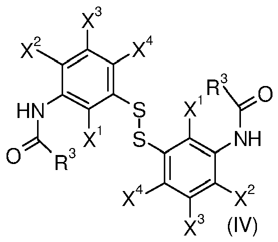 umsetzt,
(D) Disulfide der Formel (IV) mit Trifluorethylelektrophilen der Formel (IX), 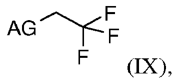 wobei AG für eine Abgangsgruppe wie zum Beispiel Chlor, Brom, Tosylat, Mesylat oder Triflat steht, in Sulfide der allgemeinen Formel (III) 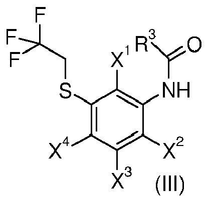 umsetzt,
(E) die Verbindungen der Formel (III) mit Diphenylchlorphosphat oder Phosphorpentachlorid zu Imidoylchloriden der allgemeinen Formel (II) umsetzt, 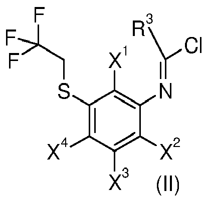
(F) die Verbindungen der allgemeinen Formel (II) mit Aminen zu Amidinen der allgemeinen Formel (Ia) 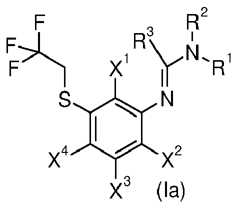 umsetzt,
(G) die Verbindungen der Formel (IA) mit Oxidationsmitteln in Sulfoxide der allgemeinen Formel (Ib) 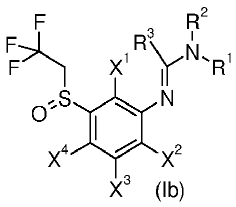
umsetzt,
wobei X¹, X², X³, X⁴, R¹, R² und R³ die oben angegebenen Bedeutungen haben.

9. Wirkstoffzusammensetzung enthaltend mindestens eine Verbindung der allgemeinen Formel (I) gemäß einem der Ansprüche 1 bis 7 und mindestens einen weiteren insektiziden, akariziden oder nematiziden Wirkstoff ausgewählt aus der Gruppe bestehend aus
(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothiophosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
Cyclodien-organochlorine, z.B. Chlordane und Endosulfan; oder
Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(IR)-trans-Isomere], Deltamethrin, Empenthrin [(EZ)-(1R)-Isomere), Esfenvalerate, Etofenprox, Fenpropathrin,
Fenvalerate, Flucythrinate, Flumethrin, tau-Fluvalinate, Halfenprox, Imiprothrin, Kadethrin, Permethrin, Phenothrin [(IR)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
DDT; oder Methoxychlor.
(4) Nikotinerge Acetylcholin-Rezeptor (nAChR) Agonisten, wie beispielsweise
Neonikotinoide, z.B. Acetamiprid, Clothianidin, Dinotefuran, Imidacloprid, Nitenpyram, Thiacloprid und Thiamethoxam; oder
Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
Avermectine/Milbemycine, z.B. Abamectin, Emamectin-benzoat, Lepimectin und Milbemectin.
(7) Juvenilhormon-Imitatoren, wie beispielsweise
Juvenilhormon-Analoge, z.B. Hydroprene, Kinoprene und Methoprene; oder
Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
Organozinnverbindungen, z.B. Azocyclotin, Cyhexatin und Fenbutatin-oxid; oder
Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
METI-Akarizide, z.B. Fenazaquin, Fenpyroximate, Pyrimidifen, Pyridaben, Tebufenpyrad und Tolfenpyrad; oder
Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
Tetron- und Tetramsäurederivate, z.B. Spirodiclofen, Spiromesifen und Spirotetramat.
(24) Komplex-IV-Elektronentransportinhibitoren, wie beispielsweise
Phosphine, z.B. Aluminiumphosphid, Calciumphosphid, Phosphin und Zinkphosphid; oder
Cyanid.
(25) Komplex-II-Elektronentransportinhibitoren, wie beispielsweise Cyenopyrafen.
(28) Ryanodinrezeptor-Effektoren, wie beispielsweise
Diamide, z.B. Chlorantraniliprole und Flubendiamide;
weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid 4-{[(6-Brompyrid-3-yl)methyl](2-fluorethyl)amino}furan-2(5H)-on (bekannt aus W02007/115644), 4-{[(6-Fluorpyrid-3-yl)methyl](2,2-difluorethyl)amino}furan-2(5H)-on (bekannt aus W02007/115644), 4-{[(2-Chlor-1,3-thiazol-5-yl)methyl](2-fluorethyl)amino}furan-2(5H)-on (bekannt aus W02007/115644), 4-{[(6-Chlorpyrid-3-yl)methyl](2-fluorethyl)amino}furan-2(5H)-on (bekannt aus W02007/115644), Flupyradifurone, 4-{[(6-Chlor-5-fluorpyrid-3-yl)methyl](methyl)amino}furan-2(5H)-on (bekannt aus W02007/115643), 4-{[(5,6-Dichlorpyrid-3-yl)methyl](2-fluorethyl)amino}furan-2(5H)-on (bekannt aus W02007/115646), 4-{[(6-Chlor-5-fluorpyrid-3-yl)methyl](cyclopropyl)amino}furan-2(5H)-on (bekannt aus W02007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus W02007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus W02007/149134) sowie Sulfoxaflor und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus W02006/089633), 3-(4'-Fluor-2,4-dimethylbiphenyl-3-yl)-4-hydroxy-8-oxa-1-azaspiro[4.5]dec-3-en-2-on (bekannt aus W02008/067911), 1-{2-Fluor-4-methyl-5-[(2,2,2-trifluorethyl)sulfinyl]phenyl}-3-(trifluormethyl)-1H-1,2,4-triazol-5-amin (bekannt aus W02006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus W02008/066153), 2-Cyan-3-(difluormethoxy)-N,N-dimethylbenzolsulfonamid (bekannt ausW02006/056433), 2-Cyan-3-(difluormethoxy)-N-methylbenzolsulfonamid (bekannt aus W02006/100288), 2-Cyan-3-(difluormethoxy)-N-ethylbenzolsulfonamid (bekannt aus W02005/035486), 4-(Difluormethoxy)-N-ethyl-N-methyl-1,2-benzothiazol-3-amin-1,1-dioxid (bekannt aus W02007/057407), N-[1-(2,3-Dimethylphenyl)-2-(3,5-dimethylphenyl)ethyl]-4,5-dihydro-1,3-thiazol-2-amin (bekannt aus W02008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus W02003/106457), 3-(2,5-Dimethylphenyl)-4-hydroxy-8-methoxy-1,8-diazaspiro[4.5]dec-3-en-2-on (bekannt aus W02009/049851), 3-(2,5-Dimethylphenyl)-8-methoxy-2-oxo-1,8-diazaspiro[4.5]dec-3-en-4-yl-ethylcarbonat (bekannt aus W02009/049851), 4-(But-2-in-1-yloxy)-6-(3,5-dimethylpiperidin-1-yl)-5-fluorpyrimidin (bekannt aus W02004/099160), (2,2,3,3,4,4,5,5-Octafluorpentyl)(3,3,3-trifluorpropyl)malononitril (bekannt aus W02005/063094), (2,2,3,3,4,4,5,5-Octafluorpentyl)(3,3,4,4,4-pentafluorbutyl)malononitril (bekannt aus W02005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus W02007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus W02007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus W02007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus W02005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus W02010/005692), NNI-0711 (bekannt aus W02002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus W02002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus W02005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus W02005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus W02005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus W02005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus W02005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus W02007/101369), 2-{6-[2-(5-Fluorpyridin-3-yl)-1,3-thiazol-5-yl]pyridin-2-yl}pyrimidin (bekannt aus W02010/006713), 2-{6-[2-(Pyridin-3-yl)-1,3-thiazol-5-yl]pyridin-2-yl}pyrimidin (bekannt aus W02010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus W02010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus W02010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus W02010/069502), N-[2-(tert-Butylcarbamoyl)-4-cyan-6-methylphenyl]-1-(3-chlorpyridin-2-yl)-3- {[5-(trifluormethyl)-2H-tetrazol-2-yl] methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus W02008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233),
und/oder mindestens einen weiteren fungiziden Wirkstoff ausgewählt aus der Gruppe bestehend aus
(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-15-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).
(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat ), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus W02010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden] amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).
(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).
(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).
(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).
(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus W02005070917).
(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).
(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).
(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).
(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus W02005042474).
(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).
(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).
(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).
(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus W02003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus W02005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus W02009094442), (15.75) 5-Fluor-2-[(4-methylbenzyl)oxy]pyrimidin-4-amin (1174376-25-0) (bekannt aus W02009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino}oxy)methyl]pyridin-2-yl}carbamat.

10. Agrochemische Zusammensetzungen, **dadurch gekennzeichnet, dass** sie mindestens eine Verbindung der Formel (I) gemäß den Ansprüchen 1 bis 7 oder eine Zusamensetzung gemäß Anspruch 9, sowie Streckmittel und/oder oberflächenaktive Stoffe enthalten.

11. Verfahren zur Herstellung agrochemischer Zusammensetzungen, **dadurch gekennzeichnet, dass** man Verbindungen der Formel (I) gemäß den Ansprüchen 1 bis 7 oder eine Zusammensetzung gemäß Anspruch 9 mit Streckmitteln und/oder oberflächenaktiven Stoffen vermischt.

12. Verfahren zur Bekämpfung tierischer Schädlinge, **dadurch gekennzeichnet, dass** man Verbindungen der Formel (I) gemäß den Ansprüchen 1 bis 7 oder eine Zusammensetzung gemäß Anspruch 9 auf tierische Schädlinge und/oder deren Lebensraum einwirken lässt.

13. Verwendung von Verbindungen der Formel (I) gemäß einem der Ansprüche 1 bis 7 oder einer Zusammensetzung gemäß Anspruch 9 zur Bekämpfung tierischer Schädlinge im Pflanzenschutz, im Materialschutz und/oder im veterinärmedizinischen Sektor.
